# EUROPÄISCHE PATENTSCHRIFT

(11) **EP 2 988 794 B1**
(45) Veröffentlichungstag und Bekanntmachung des Hinweises auf die Patenterteilung: **22.08.2018**
(21) Anmeldenummer: 14723744.0
(22) Anmeldetag: 25.04.2014
(51) Int. Cl.: A61L 27/54, A61L 29/16, A61L 31/16, C01G 39/00, C01G 39/02, C01G 41/00

(54) **VERFAHREN ZUM HERSTELLEN EINES DOTIERTEN MISCHOXIDS FÜR EINEN VERBUNDWERKSTOFF UND VERBUNDWERKSTOFF MIT EINEM SOLCHEN MISCHOXID**
METHOD FOR PRODUCING A DOPED MIXED OXIDE FOR A COMPOSITE MATERIAL, AND A COMPOSITE MATERIAL COMPRISING SUCH A MIXED OXIDE
PROCÉDÉ DE PRODUCTION D'UN OXYDE MIXTE DOPÉ POUR UN MATÉRIAU COMPOSITE ET MATÉRIAU COMPOSITE COMPRENANT LEDIT OXYDE MIXTE

(30) Priorität: 26.04.2013 DE 102013104284
(43) Veröffentlichungstag der Anmeldung: 02.03.2016
(73) Patentinhaber: Amistec GmbH & Co. KG, 6345 Kössen (AT)
(72) Erfinder: LUNK, Hans-Joachim, Towanda, Pennsylvania 18848 (US); GUGGENBICHLER, Joseph-Peter, A-6345 Kössen (AT)
(74) Vertreter: Weickmann & Weickmann PartmbB
(86) Internationale Anmeldenummer: PCT/EP2014/058472
(87) Internationale Veröffentlichungsnummer: WO 2014/174084

(56) Entgegenhaltungen:
- EP-A2- 0 264 353
- WO-A2-2008/058707
- KR-A- 20110 025 715

## Beschreibung

Die Erfindung betrifft ein Verfahren zum Herstellen eines dotierten oder undotierten Mischoxids für einen Verbundwerkstoff, einen Verbundwerkstoff mit einem solchen Mischoxid sowie Verwendungen des Mischoxids und des Verbundwerkstoffs.

Aus der WO 2008/058707 A2 ist die Verwendung von Molybdänoxiden wie beispielsweise MoO₂ und MoO₃ sowie von Wolframoxiden wie beispielsweise WO₂ und WO₃ zur Herstellung von Verbundwerkstoffen sowie zur keimfreien Ausstattung von Oberflächen bekannt. Molybdän- und Wolframoxide wandeln sich bei Kontakt mit Wasser zumindest teilweise in Molybdän- bzw. Wolframsäuren um, so dass ein saurer Oberflächen-pH-Wert ausgebildet wird, der ähnlich dem Säureschutzmantel der Haut eine antimikrobielle Wirkung erzeugt. Die Verwendung von Molybdänoxiden oder Wolframoxiden ist dabei der Verwendung anderer antimikrobiell wirksamer Substanzen wie beispielsweise Nanosilber oder organischen Bioziden überlegen, da unter anderem keine Inaktivierung der antimikrobiellen Wirksamkeit durch Schweiß oder Proteine auftritt. Weiterhin sind die genannten Oxide temperaturstabil und dadurch auch zur antimikrobiellen Ausstattung von Kunststoffen, Keramiken, Metallen und dergleichen geeignet. Schließlich weisen Molybdän- und Wolframoxide eine geringe Toxizität sowie eine gute Haut- und Schleimhautverträglichkeit auf.

Aus der EP 0 264 353 A2 sind Verbundmaterialien bekannt, die mit Calciumphosphat beschichtet sind. Die Verbundmaterialien umfassen ferner ein metallisches Substrat und eine Oxidschicht auf dem metallischen Substrat, wobei die Oxidschicht ein oder mehrere Elemente aus der Gruppe Titan, Zirkonium, Hafnium, Niob, Tantal, Zinn, Kobalt, Aluminium, Chrom, Molybdän und Wolfram enthält.

Aus der KR 2011 0025715 A sind antibakterielle und antivirale Reinigungsmittel bekannt, die einen WO₃-basierten Katalysator umfassen Aufgabe der vorliegenden Erfindung ist es, eine weiter verbesserte antimikrobielle Ausstattung von Gegenständen zu ermöglichen.

Die Aufgabe wird erfindungsgemäß durch ein Verfahren gemäß Anspruch 1 zum Herstellen eines dotierten oder undotierten Mischoxids für einen Verbundwerkstoff und durch einen Verbundwerkstoff gemäß Anspruch 9 zur Herstellung antimikrobiell wirksamer Oberflächen gelöst. Vorteilhafte Ausgestaltungen mit zweckmäßigen Weiterbildungen der Erfindung sind in den jeweiligen Unteransprüchen angegeben, wobei vorteilhafte Ausgestaltungen des Verfahrens als vorteilhafte Ausgestaltungen des Verbundwerkstoffs und umgekehrt anzusehen sind.

Ein erster Aspekt der Erfindung betrifft ein Verfahren, bei welchem ein dotiertes Mischoxid für einen Verbundwerkstoff hergestellt wird, wobei das Mischoxid die Summenformel MoₓW₁₋ₓM_{y}O_{z} besitzt, in welcher 0<x<1, 0,01≤y≤2 und 2,0≤z≤3,0 betragen und M ein von Mo und W verschiedenes Metall-Ion bezeichnet. Das erfindungsgemäße Verfahren umfasst dabei zumindest die Schritte Lösen und/oder Suspendieren wenigstens einer Molybdänverbindung und wenigstens einer Wolframverbindung in wenigstens einem flüssigen Medium, Vermischen der wenigstens einen Molybdänverbindung und der wenigstens einen Wolframverbindung in einem vorbestimmten Massenverhältnis und Trocknen der Mischung der wenigstens einen Molybdänverbindung und der wenigstens einen Wolframverbindung, wobei zum Dotieren Fremdatome M ausgewählt aus der Gruppe Na, Cu, Bi, V, Ti und Zn in das Mischoxid eingebracht werden. Im Fall von Na, Zn, Bi, Cu, Ti und V ist die Verwendung entsprechender Metallsalze besonders vorteilhaft. Besonders bevorzugt sind die Nitratsalze der genannten Elemente, weil kein störendes Anion (z.B. Cl⁻, SO4²⁻ etc.) zurückbleibt, sobald das Material getrocknet (kalziniert) wurde. Es ist aber auch möglich, reine Metalle, Legierungen oder deren Oxide zu verwenden, bevorzugt als feines Pulver. Unter einem Mischoxid werden im Rahmen der vorliegenden Erfindung "feste Lösungen" oxidischer Verbindungen verstanden, die zumindest Molybdän und Wolfram in den vorstehend genannten Molverhältnissen enthalten. Der Parameter x kann dabei grundsätzlich Werte größer 0 und kleiner 1, also beispielsweise 0,001, 0,01, 0,02, 0,03, 0,04, 0,05, 0,06, 0,07, 0,08, 0,09, 0,10, 0,11, 0,12, 0,13, 0,14, 0,15, 0,16, 0,17, 0,18, 0,19, 0,20, 0,21, 0,22, 0,23, 0,24, 0,25, 0,26, 0,27, 0,28, 0,29, 0,30, 0,31, 0,32, 0,33, 0,34, 0,35, 0,36, 0,37, 0,38, 0,39, 0,40, 0,41, 0,42, 0,43, 0,44, 0,45, 0,46, 0,47, 0,48, 0,49, 0,50, 0,51, 0,52, 0,53, 0,54, 0,55, 0,56, 0,57, 0,58, 0,59, 0,60, 0,61, 0,62, 0,63, 0,64, 0,65, 0,66, 0,67, 0,68, 0,69, 0,70, 0,71, 0,72, 0,73, 0,74, 0,75, 0,76, 0,77, 0,78, 0,79, 0,80, 0,81, 0,82, 0,83, 0,84, 0,85, 0,86, 0,87, 0,88, 0,89, 0,90, 0,91, 0,92, 0,93, 0,94, 0,95, 0,96, 0,97, 0,98, 0,99 sowie entsprechende Zwischenwerte annehmen. Der Parameter y kann grundsätzlich Werte zwischen einschließlich 0,01 und einschließlich 2, also beispielsweise 0,01, 0,02, 0,03, 0,04, 0,05, 0,06, 0,07, 0,08, 0,09, 0,10, 0,11, 0,12, 0,13, 0,14, 0,15, 0,16, 0,17, 0,18, 0,19, 0,20, 0,21, 0,22, 0,23, 0,24, 0,25, 0,26, 0,27, 0,28, 0,29, 0,30, 0,31, 0,32, 0,33, 0,34, 0,35, 0,36, 0,37, 0,38, 0,39, 0,40, 0,41, 0,42, 0,43, 0,44, 0,45, 0,46, 0,47, 0,48, 0,49, 0,50, 0,51, 0,52, 0,53, 0,54, 0,55, 0,56, 0,57, 0,58, 0,59, 0,60, 0,61, 0,62, 0,63, 0,64, 0,65, 0,66, 0,67, 0,68, 0,69, 0,70, 0,71, 0,72, 0,73, 0,74, 0,75, 0,76, 0,77, 0,78, 0,79, 0,80, 0,81, 0,82, 0,83, 0,84, 0,85, 0,86, 0,87, 0,88, 0,89, 0,90, 0,91, 0,92, 0,93, 0,94, 0,95, 0,96, 0,97, 0,98, 0,99, 1,00, 1,01, 1,02, 1,03, 1,04, 1,05, 1,06, 1,07, 1,08, 1,09, 1,10, 1,11, 1,12, 1,13, 1,14, 1,15, 1,16, 1,17, 1,18, 1,19, 1,20, 1,21, 1,22, 1,23, 1,24, 1,25, 1,26, 1,27, 1,28, 1,29, 1,30, 1,31, 1,32, 1,33, 1,34, 1,35, 1,36, 1,37, 1,38, 1,39, 1,40, 1,41, 1,42, 1,43, 1,44, 1,45, 1,46, 1,47, 1,48, 1,49, 1,50, 1,51, 1,52, 1,53, 1,54, 1,55, 1,56, 1,57, 1,58, 1,59, 1,60, 1,61, 1,62, 1,63, 1,64, 1,65, 1,66, 1,67, 1,68, 1,69, 1,70, 1,71, 1,72, 1,73, 1,74, 1,75, 1,76, 1,77, 1,78, 1,79, 1,80, 1,81, 1,82, 1,83, 1,84, 1,85, 1,86, 1,87, 1,88, 1,89, 1,90, 1,91, 1,92, 1,93, 1,94, 1,95, 1,96, 1,97, 1,98, 1,99 oder 2,00 sowie entsprechende Zwischenwerte annehmen. Der Parameter z kann grundsätzlich Werte zwischen einschließlich 2,0 und einschließlich 3,0, also beispielsweise 2,00, 2,01, 2,02, 2,03, 2,04, 2,05, 2,06, 2,07, 2,08, 2,09, 2,10, 2,11, 2,12, 2,13, 2,14, 2,15, 2,16, 2,17, 2,18, 2,19, 2,20, 2,21, 2,22, 2,23, 2,24, 2,25, 2,26, 2,27, 2,28, 2,29, 2,30, 2,31, 2,32, 2,33, 2,34, 2,35, 2,36, 2,37, 2,38, 2,39, 2,40, 2,41, 2,42, 2,43, 2,44, 2,45, 2,46, 2,47, 2,48, 2,49, 2,50, 2,51, 2,52, 2,53, 2,54, 2,55, 2,56, 2,57, 2,58, 2,59, 2,60, 2,61, 2,62, 2,63, 2,64, 2,65, 2,66, 2,67, 2,68, 2,69, 2,70, 2,71, 2,72, 2,73, 2,74, 2,75, 2,76, 2,77, 2,78, 2,79, 2,80, 2,81, 2,82, 2,83, 2,84, 2,85, 2,86, 2,87, 2,88, 2,89, 2,90, 2,91, 2,92, 2,93, 2,94, 2,95, 2,96, 2,97, 2,98, 2,99 oder 3,00 sowie entsprechende Zwischenwerte annehmen. Die Herstellung derartiger Mischoxide ist insbesondere aufgrund der ähnlichen Atomradien von Molybdän und Wolfram möglich. Die Erfinder haben festgestellt, dass die antimikrobielle Wirksamkeit von derartigen Mischoxiden stärker ausgeprägt ist, als es ein rein additiver Effekt von Mischungen aus Molybdänoxid und Wolframoxiden erklären würde. Darüber hinaus besitzen die erfindungsgemäß hergestellten Mischoxide eine deutlich geringere Wasserlöslichkeit als die entsprechenden Molybdän- und Wolframoxide, wodurch ihre antimikrobielle Wirkung auch in feuchten Umgebungen sowie bei Anwendungen unter Wasser besonders lange aufrechterhalten wird. In Abhängigkeit der verwendeten Molybdän- und Wolframverbindungen werden derartige Mischoxide in einfachster Ausgestaltung dadurch hergestellt, dass zunächst entsprechende Lösungen und/oder Suspensionen der entsprechenden Molybdän- und Wolframverbindung(en) hergestellt werden. Die Lösungen bzw. Suspensionen können dabei grundsätzlich getrennt voneinander hergestellt werden. Alternativ oder zusätzlich können in einer einzelnen Lösung/Suspension mehrere oder alle verwendeten Molybdän- und/oder Wolframverbindungen enthalten sein. Unter einem flüssigen Medium sind im Rahmen der Erfindung Reinstoffe oder Reinstoffmischungen zu verstehen, die zumindest unter SATP-Bedingungen (Standard Ambient Temperature and Pressure, T=298,15 K (25 °C), p=101.300 Pa) flüssig sind. Das Vermischen der verwendeten Molybdän- und Wolframverbindungen kann ebenfalls gleichzeitig mit der Herstellung der Lösung/Suspension erfolgen, beispielsweise indem die verwendeten Molybdän- und Wolframverbindungen gemeinsam in einen entsprechenden Behälter mit dem flüssigen Medium gegeben werden. Alternativ oder zusätzlich können zunächst Lösungen/Suspensionen einzelner oder mehrerer Molybdän- und/oder Wolframverbindungen hergestellt und anschließend im gewünschten Verhältnis miteinander vermischt werden, um das gewünschte Massen- bzw. Molverhältnis der einzelnen Komponenten einzustellen. In Abhängigkeit der Ausgestaltung der Komponente M, die wenigstens ein von Mo und W verschiedenes Metall-Ion aus der Gruppe Na, Cu, Bi, V, Ti und Zn bezeichnet, können grundsätzlich Molybdän- und/oder Wolframverbindungen verwendet werden, die die Komponente M bereits enthalten. Alternativ oder zusätzlich können eine oder mehrere weitere Verbindungen zu den Lösung(en)/Suspension(en) zugegeben werden, die die Komponente M bereitstellen. Anschließend wird das flüssige Medium entfernt, wodurch das Mischoxid der genannten Summenformel MoₓW₁₋ₓM_{y}O_{z} erhältlich ist bzw. erhalten wird. Unter einer Dotierung wird im Rahmen der vorliegenden Erfindung das Einbringen von Fremdatomen in das Mischoxid verstanden, wobei alle Fremdatome zusammen maximal 10 Mol-% des Mischoxids, also beispielsweise 10,0 Mol-%, 9,9 Mol-%, 9,8 Mol-%, 9,7 Mol-%, 9,6 Mol-%, 9,5 Mol-%, 9,4 Mol-%, 9,3 Mol-%, 9,2 Mol-%, 9,1 Mol-%, 9,0 Mol-%, 8,9 Mol-%, 8,8 Mol-%, 8,7 Mol-%, 8,6 Mol-%, 8,5 Mol-%, 8,4 Mol-%, 8,3 Mol-%, 8,2 Mol-%, 8,1 Mol-%, 8,0 Mol-%, 7,9 Mol-%, 7,8 Mol-%, 7,7 Mol-%, 7,6 Mol-%, 7,5 Mol-%, 7,4 Mol-%, 7,3 Mol-%, 7,2 Mol-%, 7,1 Mol-%, 7,0 Mol-%, 6,9 Mol-%, 6,8 Mol-%, 6,7 Mol-%, 6,6 Mol-%, 6,5 Mol-%, 6,4 Mol-%, 6,3 Mol-%, 6,2 Mol-%, 6,1 Mol-%, 6,0 Mol-%, 5,9 Mol-%, 5,8 Mol-%, 5,7 Mol-%, 5,6 Mol-%, 5,5 Mol-%, 5,4 Mol-%, 5,3 Mol-%, 5,2 Mol-%, 5,1 Mol-%, 5,0 Mol-%, 4,9 Mol-%, 4,8 Mol-%, 4,7 Mol-%, 4,6 Mol-%, 4,5 Mol-%, 4,4 Mol-%, 4,3 Mol-%, 4,2 Mol-%, 4,1 Mol-%, 4,0 Mol-%, 3,9 Mol-%, 3,8 Mol-%, 3,7 Mol-%, 3,6 Mol-%, 3,5 Mol-%, 3,4 Mol-%, 3,3 Mol-%, 3,2 Mol-%, 3,1 Mol-%, 3,0 Mol-%, 2,9 Mol-%, 2,8 Mol-%, 2,7 Mol-%, 2,6 Mol-%, 2,5 Mol-%, 2,4 Mol-%, 2,3 Mol-%, 2,2 Mol-%, 2,1 Mol-%, 2,0 Mol-%, 1,9 Mol-%, 1,8 Mol-%, 1,7 Mol-%, 1,6 Mol-%, 1,5 Mol-%, 1,4 Mol-%, 1,3 Mol-%, 1,2 Mol-%, 1,1 Mol-%, 1,0 Mol-%, 0,9 Mol-%, 0,8 Mol-%, 0,7 Mol-%, 0,6 Mol-%, 0,5 Mol-%, 0,4 Mol-%, 0,3 Mol-%, 0,2 Mol-%, 0,1 Mol-% oder 0 Mol-% ausmachen. Weiterhin kann vorgesehen sein, dass im Rahmen des erfindungsgemäßen Verfahrens mehrere unterschiedliche Mischoxide bzw. ein heterogenes Mischoxid mit innerhalb der angegebenen Summenformel variierenden Bestandteilen hergestellt werden.

In einer vorteilhaften Ausgestaltung der Erfindung ist vorgesehen, dass die wenigstens eine Molybdänverbindung ausgewählt wird aus einer Gruppe, die Ammoniumdimolybdat ((NH₄)₂Mo₂O₇, ADM), Ammoniumparamolybdat (APM), Ammoniumpentamolybdat, Ammoniumheptamolybdat, Molybdänsäure, Molybdänoxidhydrate, Molybdänoxid, Molybdänsuboxid, metallisches Molybdän und Polyoxomolybdate umfasst und/oder dass die wenigstens eine Wolframverbindung ausgewählt wird aus einer Gruppe, die Ammoniummetawolframate ((NH₄)₆[α-H₂W₁₂O₄₀] * 3 H₂O, AMT), Wolframsäure, Wolframoxidhydrate, Wolframoxid, Wolframsuboxid, metallisches Wolfram und Polyoxowolframate umfasst. Durch Verwendung einer oder mehrerer der genannten Verbindungen ist es möglich, das Mischoxid besonders schnell, einfach und mit einer besonders präzisen Zusammensetzung herzustellen. Die Verwendung einer oder mehrerer der genannten Verbindungen bietet den zusätzlichen Vorteil, dass eine hohe Oxidbildung sichergestellt ist und das Mischoxid somit mit besonders hohen Ausbeuten erhalten wird.

Weitere Vorteile ergeben sich, wenn das Mischoxid mit einer Fluor-Verbindung, insbesondere mit einem Oxyfluorid, WOF₄, WO₂F₂, Calciumfluorid und/oder Fluorapatit, dotiert wird. Eine Dotierung, das heißt die Zugabe von Fremdatomen, die nicht unter die Definition des Parameters M fallen, mit einer Fluor-Verbindung bietet den zusätzlichen Vorteil, dass die Adhäsion von Mikroorganismen an der Oberfläche des Mischoxids zusätzlich erschwert wird. Dies verhindert somit die Besiedelung von mit dem Mischoxid versehenen Oberflächen und verbessert damit den antimikrobiellen Effekt zusätzlich. Vorzugsweise werden hierbei Fluor-Verbindungen mit einer möglichst geringen Wasserlöslichkeit verwendet, um ein Auswaschen zu verhindern oder zumindest zu verlangsamen. Nicht abschließende Beispiele für geeignete Verbindungen sind Calciumfluorid (CaF₂) und Fluorapatit (Ca₅[F|(PO₄)₃]). Die Verwendung von WOF₄, WO₂F₂ und/oder korrespondierenden Molybdänoxyfluoriden bietet den zusätzlichen Vorteil, dass diese gleichzeitig zur Herstellung des Mischoxids und zu dessen Dotierung mit Fluorid-Ionen bzw. Fluor-Verbindungen beitragen. Zusätzlich zur Dotierung der Mischoxide mit Na, Zn, Bi, Cu, Ti und V ist es grundsätzlich möglich, Oxide von Zn, Bi, Cu, Ti und/oder V gemeinsam mit einem oder mehreren Mischoxiden einzusetzen, insbesondere die Verbindungen TiO₂, ZnO und V₂O₅.

In einer weiteren vorteilhaften Ausgestaltung der Erfindung ist vorgesehen, dass das flüssige Medium ausgewählt wird aus einer Gruppe, die unpolare und/oder polare und/oder protische und/oder aprotische Lösungsmittel umfasst. Das flüssige Medium kann damit optimal an die jeweils verwendeten Molybdän- und Wolframverbindungen angepasst werden. Grundsätzlich eignen sich beispielsweise polare protische oder aprotische Medien wie Wasser, Acetonitril oder Alkohole. Vor allem Methylalkohole haben sich als besonders vorteilhaft herausgestellt. Aber auch unpolare Kohlenwasserstoffe sind grundsätzlich geeignet und können besonders einfach wieder abgezogen bzw. entfernt werden.

In einer weiteren vorteilhaften Ausgestaltung der Erfindung ist vorgesehen, dass Lösungen und/oder Suspensionen der wenigstens einen Molybdänverbindung und der wenigstens einen Wolframverbindung mit Hilfe wenigstens eines Verfahrens aus der Gruppe Sprühtrocknen, Gefriertrocknen, Verbrennungssynthese, Flammenhydrolyse, Gasphasensynthese und/oder Spraypyrolyse getrocknet werden. Die Lösung(en)/Suspension(en) können beispielsweise verdüst werden. Manche Ausgangsstoffe bilden aber beim Vereinigen ihrer Lösungen eine Trübung bzw. einen Niederschlag, beispielsweise Zinknitrat und ADM. In einem solchen Fall ist ein separates Verdüsen dieser Stoffe vorzuziehen. Die Bildung der Mischoxide kann aber grundsätzlich nach unterschiedlichen Verfahren erfolgen. Sprühtrocknung ist besonders vorteilhaft. Ebenfalls bewährt hat sich Gefriertrocknung. Auch möglich sind die Verfahren Verbrennungssynthese, Flammenhydrolyse, Gasphasensynthese, und Spraypyrolyse. Flammenbasierte Verfahren haben den Vorteil, besonders feine Teilchen zu erzeugen, die mittlere Korngrößen im Bereich bis zu wenigen nm aufweisen können. Zur Suspension der Vorläufersubstanzen verwendete Kohlenwasserstoffe können dabei vorteilhaft als Energieträger verwendet werden. Das Mischoxid bzw. die Mischoxide fallen je nach Trocknungsverfahren in unterschiedlicher Korngrößenverteilung und mit unterschiedlichen Restfeuchtigkeitswerten an.

Weitere Vorteile ergeben sich, indem nach dem Trocknen wenigstens ein Kalzinierungsschritt und/oder wenigstens ein Zerkleinerungsschritt durchgeführt wird bzw. werden. Unter einer Kalzinierung ist eine Wärmebehandlung zu verstehen. Die Kalzinierung kann beispielsweise in einem Festbett oder in einer Wirbelschicht erfolgen, bei Verweilzeiten von wenigen Sekunden bis zu mehreren Stunden. Durch eine Kalzinierung kann in Abhängigkeit der verwendeten Molybdän- und Wolframverbindungen neben einer Entwässerung bedarfsweise auch eine teilweise oder vollständige Oxidation bzw. Oxidbildung erzielt werden. Im Festbett sind unterschiedliche Schichtdicken möglich. Durch einen Zerkleinerungsschritt kann die Oberfläche des Mischoxids und damit seine antimikrobielle Wirksamkeit vorteilhaft erhöht werden. Der Kalzinierungsschritt und der Zerkleinerungsschritt können grundsätzlich unabhängig voneinander einmal oder mehrmals in beliebiger Reihenfolge durchgeführt werden.

Dabei hat es sich in weiterer Ausgestaltung der Erfindung als vorteilhaft gezeigt, wenn der Kalzinierungsschritt unter oxidierender und/oder reduzierender Atmosphäre und/oder unter Schutzgas und/oder bei Temperaturen zwischen 150°C und 1000°C durchgeführt wird. Das Kalzinieren hat Einfluss auf die spezifische Oberfläche des Mischoxids sowie auf die darin enthaltenden Fehlstellen. Fehlstellen bilden sich vor allem in sauerstoffdefizitären Verbindungen und vor allem bei Vorliegen der monoklinen Kristallstruktur im Mischoxid. Grundsätzlich kann gesagt werden, dass höhere Kalzinierungstemperaturen zu geringeren spezifischen Oberflächen führen. Bei höheren Kalzinierungstemperaturen werden teilweise Fehlstellen im Mischoxid abgebaut, teilweise aber auch neue geschaffen. Unter einer Temperatur zwischen 150°C und 1000°C sind insbesondere Temperaturen von 150°C, 170°C, 190°C, 210°C, 230°C, 250°C, 270°C, 290°C, 310°C, 330°C, 350°C, 370°C, 390°C, 410°C, 430°C, 450°C, 470°C, 490°C, 510°C, 530°C, 550°C, 570°C, 590°C, 610°C, 630°C, 650°C, 670°C, 690°C, 710°C, 730°C, 750°C, 770°C, 790°C, 810°C, 830°C, 850°C, 870°C, 890°C, 910°C, 930°C, 950°C, 970°C, 990°C oder 1000°C sowie entsprechende Zwischentemperaturen wie beispielsweise 150°C, 151°C, 152°C, 153°C, 154°C, 155°C, 156°C, 157°C, 158°C, 159°C, 160°C, 116°C, 162°C, 163°C, 164°C, 165°C, 166°C, 167°C, 168°C, 169°C, 170°C und so weiter zu verstehen. Üblicherweise hat sich eine Kalzinierungstemperatur im Bereich von 200°C bis 400°C und/oder eine Verweilzeit von 0,5 bis 4 Stunden als vorteilhaft gezeigt. Alternativ oder zusätzlich kann vorgesehen sein, dass der Zerkleinerungsschritt durch Trockenmahlung und/oder durch Strahlvermahlen und/oder bis zu einer mittleren Korngröße des Mischoxids von 0,1 µm bis 200 µm durchgeführt wird. Unter einer mittleren Korngröße von 0,1 µm bis 200 µm werden insbesondere Korngrößen von 0,1 µm, 5 µm, 10 µm, 15 µm, 20 µm, 25 µm, 30 µm, 35 µm, 40 µm, 45 µm, 50 µm, 55 µm, 60 µm, 65 µm, 70 µm, 75 µm, 80 µm, 85 µm, 90 µm, 95 µm, 100 µm, 105 µm, 110 µm, 115 µm, 120 µm, 125 µm, 130 µm, 135 µm, 140 µm, 145 µm, 150 µm, 155 µm, 160 µm, 165 µm, 170 µm, 175 µm, 180 µm, 185 µm, 190 µm, 195 µm und 200 µm sowie entsprechende Zwischenwerte wie beispielsweise 0,1 µm, 0,2 µm, 0,3 µm, 0,4 µm, 0,5 µm, 0,6 µm, 0,7 µm, 0,8 µm, 0,9 µm, 1,0 µm, 1,1 µm, 1,2 µm, 1,3 µm, 1,4 µm, 1,5 µm, 1,6 µm, 1,7 µm, 1,8 µm, 1,9 µm, 2,0 µm, 2,1 µm, 2,2 µm, 2,3 µm, 2,4 µm, 2,5 µm, 2,6 µm, 2,7 µm, 2,8 µm, 2,9 µm, 3,0 µm, 3,1 µm, 3,2 µm, 3,3 µm, 3,4 µm, 3,5 µm, 3,6 µm, 3,7 µm, 3,8 µm, 3,9 µm, 4,0 µm, 4,1 µm, 4,2 µm, 4,3 µm, 4,4 µm, 4,5 µm, 4,6 µm, 4,7 µm, 4,8 µm, 4,9 µm, 5,0 µm, 5,1 µm, 5,2 µm, 5,3 µm, 5,4 µm, 5,5 µm, 5,6 µm, 5,7 µm, 5,8 µm, 5,9 µm, 6,0 µm, 6,1 µm, 6,2 µm, 6,3 µm, 6,4 µm, 6,5 µm, 6,6 µm, 6,7 µm, 6,8 µm, 6,9 µm, 7,0 µm, 7,1 µm, 7,2 µm, 7,3 µm, 7,4 µm, 7,5 µm, 7,6 µm, 7,7 µm, 7,8 µm, 7,9 µm, 8,0 µm, 8,1 µm, 8,2 µm, 8,3 µm, 8,4 µm, 8,5 µm, 8,6 µm, 8,7 µm, 8,8 µm, 8,9 µm, 9,0 µm, 9,1 µm, 9,2 µm, 9,3 µm, 9,4 µm, 9,5 µm, 9,6 µm, 9,7 µm, 9,8 µm, 9,9 µm, 10,0 µm und so weiter verstanden. Die Korngröße bzw. Korngrößenverteilung kann grundsätzlich durch Laserbeugung bzw. Laserstreuung ermittelt werden. Als besonders vorteilhaft haben sich üblicherweise Korngrößen ≤5 µm erwiesen. Die Mischoxide können auch als Agglomerate der einzelnen Körner vorliegen. Die Korngröße kann auch indirekt über die spezifische Oberfläche, z. B. nach BET, ermittelt werden. Typische Werte sind 0,5 bis 5 m²/g. Es haben sich aber auch Mischoxide mit deutlich größerer und deutlich kleinerer spezifischer Oberfläche als antimikrobiell wirksam herausgestellt.

Weitere Vorteile ergeben sich, indem das wenigstens eine Mischoxid zum Herstellen eines Verbundwerkstoffs in wenigstens einen weiteren Werkstoff eingearbeitet und/oder auf die Oberfläche des wenigstens einen weiteren Werkstoffs aufgebracht wird. Hierdurch kann die antimikrobielle Wirkung besonders flexibel für unterschiedlichste Anwendungszwecke bereitgestellt werden. Unter einem Verbundwerkstoff werden im Rahmen der Erfindung Werkstoff aus zwei oder mehr verbundenen Materialien verstanden, wobei das wenigstens eine Mischoxid wenigstens eines der Materialien darstellt. Ein Verbundwerkstoff besitzt andere Werkstoffeigenschaften als seine einzelnen Komponenten. Für die Eigenschaften des Verbundwerkstoffs sind stoffliche Eigenschaften und Geometrien der einzelnen Komponenten von Bedeutung. Die Form der Mischoxide kann unter anderem sphärisch, zylindrisch, plättchenförmig und/oder faserig sein. Sie ergibt sich unter anderem aus dem eingesetzten Zerkleinerungsverfahren. Die Verbindung der Werkstoffe erfolgt bevorzugt durch Stoff- oder Formschluss oder eine Kombination von beidem. Vor allem Polymere, Silikone, Kunststoffe, Farben, Lacke und Keramiken lassen sich mit dem Mischoxid antimikrobiell ausstatten. Typischerweise werden zwischen 0,5 und 5 % (Masse) des oder der Mischoxide in das oder die betreffenden Materialien eingebracht. Gängige Verfahren sind beispielsweise Eincompoundieren für Kunststoffe (Extruder) und Einschneiden für Farben und Lacke (Dissolver). Es ist möglich, 0,1 bis 80% (Masse) des oder der Mischoxide in einen Träger einzubringen, entweder direkt als Endprodukt oder als Konzentrat für die Weiterverarbeitung (sogenanntes Masterbatch).

Ein zweiter Aspekt der Erfindung betrifft einen Verbundwerkstoff zur Herstellung antimikrobiell wirksamer Oberflächen, wobei der Verbundwerkstoff erfindungsgemäß wenigstens ein dotiertes Mischoxid enthält. Das Mischoxid besitzt die Summenformel MoₓW₁₋ₓM_{y}O_{z}, in welcher 0<x<1, 0,01≤y≤2 und 2,0≤z≤3,0 betragen und M ein von Mo und W verschiedenes Metall-Ion bezeichnet, wobei als Dotierung Fremdatome M aus der Gruppe Na, Cu, Bi, V, Ti und Zn in das Mischoxid eingebracht sind. Der erfindungsgemäße Verbundwerkstoff ermöglicht somit eine verbesserte antimikrobielle Ausstattung von Gegenständen, die teilweise oder vollständig aus dem Verbundwerkstoff bestehen bzw. aus diesem hergestellt sind. Weitere Merkmale und deren Vorteile sind der Beschreibung des ersten Erfindungsaspekts zu entnehmen, wobei vorteilhafte Ausgestaltungen des ersten Erfindungsaspekts als vorteilhafte Ausgestaltungen des zweiten Erfindungsaspekts und umgekehrt anzusehen sind. Der erfindungsgemäße Verbundwerkstoff kann grundsätzlich frei von elementarem Molybdän, MoO₂, MoO₃, Molybdänkarbid, Molybdännitrid, Molybdänsilizid oder Molybdänsulfid, Molybdänhexacarbonyl, Molybdänacetylacetonat und/oder Molybdän-haltigen Legierungen ausgebildet sein. Gleiches gilt für elementares Wolfram und die korrespondierenden Wolfram-Verbindungen und -Legierungen. Ebenso kann der Verbundwerkstoff grundsätzlich frei von nicht-säurebildenden Metalloxiden wie Zinkoxid, Titanoxid, Titandioxid, Aluminiumoxid oder sonstigen nicht-säurebildenden Photokatalysatoren ausgebildet sein. Weiterhin kann der Verbundwerkstoff grundsätzlich ohne die Verwendung zusätzlicher antimikrobiell wirksamer Verbindungen wie beispielsweise Silber, insbesondere Nanosilber, bzw. Silberverbindungen, insbesondere löslicher Silberverbindungen wie Silbernitrat und dergleichen, Kupfer, organischen Biozide, Zeolithen oder dergleichen ausgebildet sein, wodurch neben einer besseren Umweltverträglichkeit des erfindungsgemäß hergestellten Verbundwerkstoffs auch erhebliche Kostensenkungen und eine höhere Beständigkeit gegeben sind.

In einer vorteilhaften Ausgestaltung der Erfindung ist vorgesehen, dass x zwischen 0,50 und 0,70 beträgt und/oder dass y zwischen 0,01 und 0,10 beträgt und/oder dass z zwischen 2,50 und 3,0 beträgt und/oder dass ein molares W:Mo-Verhältnis zwischen 250:1 und 1:250, insbesondere zwischen 3:1 und 1:3 beträgt. Hierdurch ist das wenigstens eine Mischoxid und damit der Verbundwerkstoff besonders stark antimikrobiell wirksam.

In einer weiteren vorteilhaften Ausgestaltung der Erfindung enthält der Verbundwerkstoff bezogen auf sein Gesamtgewicht einen Massenanteil zwischen 0,01 % und 80 %, insbesondere zwischen 0,1 % und 10 % an Mischoxid. Beispielsweise kann der Verbundwerkstoff also einen Anteil von 0,01 %, 0,1 %, 1 %, 2 %, 3 %, 4 %, 5 %, 6 %, 7 %, 8 %, 9 %, 10 %, 11 %, 12 %, 13 %, 14 %, 15 %, 16 %, 17 %, 18 %, 19 %, 20 %, 21 %, 22 %, 23 %, 24 %, 25 %, 26 %, 27 %, 28 %, 29 %, 30 %, 31 %, 32 %, 33 %, 34 %, 35 %, 36 %, 37 %, 38 %, 39 %, 40 %, 41 %, 42 %, 43 %, 44 %, 45 %, 46 %, 47 %, 48 %, 49 %, 50 %, 51 %, 52 %, 53 %, 54 %, 55 %, 56 %, 57 %, 58 %, 59 %, 60 %, 61 %, 62 %, 63 %, 64 %, 65 %, 66 %, 67 %, 68 %, 69 %, 70 %, 71 %, 72 %, 73 %, 74 %, 75 %, 76 %, 77 %, 78 %, 79 % oder 80 % bzw. entsprechende Zwischenwerte von beispielsweise 0,01 %, 0,1 %, 0,2 %, 0,3 %, 0,4 %, 0,5 %, 0,6 %, 0,7 %, 0,8 %, 0,9 %, 1,0 % und so weiter enthalten. Hierdurch kann die antimikrobielle Wirksamkeit optimal an den jeweiligen Einsatzzweck des Verbundwerkstoffs angepasst werden.

Eine besonders hohe antimikrobielle Wirksamkeit wird in weiterer Ausgestaltung der Erfindung dadurch erzielt, dass das wenigstens eine Mischoxid in Form von Partikeln mit einem mittleren Durchmesser zwischen 0,1 µm und 200 µm, insbesondere zwischen 0,5 µm und 10 µm vorliegt.

Weiterhin kann es erfindungsgemäß vorgesehen sein, dass der Verbundwerkstoff wenigstens einen weiteren Werkstoff umfasst, der ausgewählt ist aus einer Gruppe, die organische und anorganische Polymere, Kunststoffe, Silikone, Keramiken, Gummi, Pulverlacke, Flüssiglacke, Bitumen, Asphalt, Gläser, Wachse, Harze, Farben, Textilien, Gewebe, Holz, Kompositwerkstoffe, Metalle und Hydrophilierungsmittel umfasst. Auf diese Wiese kann der erfindungsgemäße Verbundwerkstoff besonders variabel ausgestaltet und für unterschiedlichste Anwendungszwecke verwendet werden. Als organische Polymere sind insbesondere thermoplastische Polymere wie beispielsweise Polypropylen (PP), Polyethylen (PE), Polyethylenterephthalat (PET), Polyvinylchlorid (PVC), Polystyrol (PS), Polycarbonat (PC), ein Poly(meth)acrylat (z. B. PAA, PAN, PMA, PBA, ANBA, ANMA, PMMA, AMMA, MABS und/oder MBS), Acrylnitril-Butadien-Styrol (ABS), Polyurethane (PU), thermoplastische Polyurethane (TPU), Thermoplastische Elastomere (TPE), thermoplastisch Vulkanisate (TPV), Polyoxymethylen (POM), Polyethylenterephthalat (PET), Epoxidharze, Melamine und Polymilchsäure zu nennen. Aber auch duroplastische Polymere können als Matrix und/oder Trägermaterial für das Mischoxid vorgesehen sein. Als keramische Werkstoffe sind im besonderen anorganische Formulierungen, die die Stoffe Aluminiumoxid, Titanoxid, Siliziumoxid, Siliziumkarbid und/oder Zirkonoxid enthalten, geeignet. Um auf die üblichen Herstellmethoden und Bedingungen für Keramiken zurückgreifen zu können, eignen sich Mischoxide, die in der höchsten Oxidationsstufe vorliegen, das heißt bei denen der Parameter z zwischen etwa 2,8 und 3,0 beträgt. Ein Hydrophilierungsmittel steigert die Benetzbarkeit der Oberfläche des Verbundwerkstoffs mit Wasser im Vergleich zur Benetzbarkeit der Oberfläche des Verbundwerkstoffs ohne Zusatz des Hydrophilierungsmittels. Dies kann beispielsweise über den Kontaktwinkel eines Wassertropfens auf der Oberfläche des Verbundwerkstoffs gemessen werden. Es hat sich überraschender Weise herausgestellt, dass die antimikrobielle Wirksamkeit im Gegensatz zur bisher vorherrschenden Ansicht in der Fachwelt vorteilhaft gesteigert werden kann, indem der Verbundwerkstoff nicht möglichst hydrophob, sondern im Gegenteil durch Zugabe eines Hydrophilierungsmittels hydrophil ausgebildet ist. Dabei kann grundsätzlich sogar vorgesehen sein, dass der Verbundwerkstoff zumindest im Bereich seiner Oberfläche gegebenenfalls leicht hygroskopisch ist. Unter hygroskopisch ist zu verstehen, dass der Verbundwerkstoff zumindest an seiner Oberfläche bzw. in den oberflächennahen Bereichen Feuchtigkeit aufnimmt. Beispielsweise sollte der Verbundwerkstoff in Umgebungen mit 10% relativer Luftfeuchtigkeit zwischen 0,01 bis 10 Gew.-% Feuchtigkeit aufnehmen. Besonders vorteilhaft sind 0,1 bis 3% Gleichgewichtsfeuchtigkeit, die sich in der Regel nach einigen Minuten bis Stunden einstellen. Die Zugabe des Hydrophilierungsmittels setzt die Oberflächenspannung des Verbundwerkstoffs herab und erzeugt somit eine hydrophilere bzw. hygroskopischere Oberfläche des Verbundwerkstoffs. Die Erfindung beruht dabei auf der Erkenntnis, dass eine verringerte antimikrobielle Wirksamkeit besonders in hydrophoben Verbundwerkstoffen auftritt, da diese apolaren Verbundwerkstoffe keine oder nur sehr wenig Feuchtigkeit an ihrer Oberfläche enthalten bzw. binden können. Demgegenüber ermöglicht der erfindungsgemäße Verbundwerkstoff eine verbesserte Benetzung seiner Oberfläche mit Wasser, so dass sich in Abhängigkeit des jeweiligen Agens mehr antimikrobiell wirksame Metalle, Metallionen und/oder Metallverbindungen bilden können bzw. freigesetzt werden können. Indem der erfindungsgemäße Verbundwerkstoff neben dem wenigstens einen Mischoxid ein oder mehrere Hydrophilierungsmittel umfasst, kann somit einerseits die antimikrobielle Wirksamkeit des Mischoxids verstärkt und andererseits die Menge des eingesetzten Mischoxids um bis zu 95% bei gleicher oder besserer antimikrobieller Wirksamkeit gesenkt werden. Hierdurch ergeben sich erhebliche Kosteneinsparungen sowie verschiedene weitere Vorteile, da die Menge der im Verbundwerkstoff enthaltenen bzw. vom Verbundwerkstoff freigesetzten Metalle bzw. Metallverbindungen ohne Wirkungsverlust vorteilhaft gesenkt werden kann.

Obwohl grundsätzlich eine kovalente Anbindung des Hydrophilierungsmittels an einen als Trägermittel dienenden weiteren Werkstoff denkbar ist, liegt bzw. liegen das oder die Hydrophilierungsmittel bevorzugt nicht kovalent gebunden im Verbundwerkstoff vor, sondern ist bzw. sind mit einem Trägermittel vermischt. Die Mischung aus Trägermittel und Hydrophilierungsmittel kann dabei grundsätzlich homogen bzw. einphasig oder heterogen bzw. mehrphasig sein. Der erfindungsgemäße Verbundwerkstoff eignet sich auch für verschiedene Einsatzzwecke, für welche die aus dem Stand der Technik bekannten Verbundmaterialien bislang nicht verwendet werden konnten. Geeignete Hydrophilierungsmittel sind beispielsweise migrierende Additive, insbesondere Glyzerinmonostearat, Alginate, Kollagen, Chitosan, Gelatine, Polyethylenglykol (PEG), Polyethylenglykolester, Polypropylenglykol (PPG), Polypropylenglykolester, Polycarboxylate, Polyacrylsäuren, Polysaccaride, insbesondere Stärke und/oder thermoplastische Stärke, Polymilchsäure (PLA), Humussäuren, Lignin, Maleinsäure, Erucasäure, Ölsäure, Stearate, Kiegelgel, insbesondere fumed silica und/oder Zeolithe, Melasse, Polydextrose, Metallhydroxide, insbesondere Al(OH)₃ und/oder Mg(OH)₂, Aluminiumoxid, insbesondere fused alumina, Copolymere mit Acrylsäure, insbesondere Copolymerisate aus Polystyrol und Acrylsäure, Säureanhydride, insbesondere P₄O₁₀, und Glykosaminoglykane, insbesondere Heparin umfasst. Ebenso haben sich Alkylaminalkoholate, DMMB (Dimethylmethylenblau) sowie weitere Methylenblau-Derivate bewährt. Besonders gut funktionieren unter anderem auch organische Säuren wie Behensäure oder Isophthalsäure, Antistatika, Anti-Fogging-Mittel, Gleitmittel, Polyalkohole, Gelatine, Glyzerin, alkylierte Amine, alkylierte Alkoxyamine und Glyzerinmonostearate. Alkylierte Ethoxyamine sind nach bisherigen Erkenntnissen am besten geeignet.

In einer weiteren vorteilhaften Ausgestaltung der Erfindung ist vorgesehen, dass der Verbundwerkstoff zusätzlich zum dotierten Mischoxid wenigstens ein Molybdat und/oder wenigstens ein Wolframat und/oder eine Verbindung der Summenformel Aⁿ⁺_{z}MO₄, in welcher M Mo und/oder W bedeutet, A wenigstens ein von Mo und W verschiedenes Metall-Ion und/oder NH₄⁺ bezeichnet und n*z=+2 beträgt, enthält. Durch die Verwendung einer oder mehrerer der genannten Verbindungen wird überraschenderweise neben einer guten antimikrobiellen Wirksamkeit auch eine besonders hohe Lichtstabilität, insbesondere gegenüber UV-Licht, erzielt. Somit wird das Auftreten unerwünschter Verfärbungen an der Oberfläche des erfindungsgemäß hergestellten Verbundwerkstoffs bzw. eines daraus hergestellten Bauteils besonders zuverlässig verhindert. Darüber hinaus weisen derartige Molybdate und Wolframate insbesondere in Verbundwerkstoffen eine besonders geringe Wasserlöslichkeit auf und sind zumindest im Wesentlichen farblos bzw. weiß. Hierdurch eignet sich der erfindungsgemäße Verbundwerkstoff besonders gut zur Herstellung von Gegenständen, für die eine neutrale, weiße Oberfläche gewünscht ist. Umgekehrt kann aufgrund der neutral-weißen Farbe der Oberfläche aber auch eine einfache Einfärbung durch Zusatz entsprechender Farbstoffe bzw. Farbpigmente vorgenommen werden. Jedes Molybdat bzw. Wolframat kann grundsätzlich in einer Menge zwischen 0,1 Gew.-% und 80 Gew.-% im Verbundwerkstoff vorliegen. Vorzugsweise besitzt ein aus dem Verbundwerkstoff hergestelltes Endprodukt zumindest im Bereich seiner Oberfläche zwischen 0,5 Gew.-% und 5 Gew.-% Molybdate/Wolframate.

In weiterer Ausgestaltung der Erfindung hat es sich als vorteilhaft gezeigt, wenn A der Summenformel Aⁿ⁺_{z}MO₄ ausgewählt wird aus einer Gruppe, die Na, K, Mg, Ca, Ag, Cu, Bi, V, Ti und Zn umfasst. Hierdurch können die Löslichkeit, die Farbe und die antimikrobielle Wirksamkeit des Verbundwerkstoffs optimal an seinen jeweiligen Einsatzzweck angepasst werden. Mit Hilfe der genannten Verbindungen, einzeln und in beliebiger Kombination, kann zudem die Adhäsion von Mikroorganismen an der Oberfläche des Verbundwerkstoffs zusätzlich erschwert werden. Dies verhindert besonders effektiv die Besiedelung der Oberfläche des Verbundwerkstoffs. Die genannten Molybdate und/oder Wolframate können besonders schnell, einfach und kostengünstig durch gemeinsames Erhitzen der entsprechenden Carbonate, beispielsweise Na₂CO₃, ZnCO₃, CaCO₃ usw., mit MoO₃ und/oder WO₃ im Rahmen einer Feststoffsynthese hergestellt werden. Alternativ können die genannten Molybdate und/oder Wolframate durch Eintropfen von Lösungen entsprechender Nitrate, beispielsweise von ZnNO₃, Ag₂NO₃, CuNO₃ etc., in Na₂MoO₄- bzw. Na₂WO₄-Lösungen und Abtrennen der ausgefällten Reaktionsprodukte hergestellt werden.

Es kann vorgesehen sein, dass der Verbundwerkstoff zusätzlich zum dotierten und/oder undotierten Mischoxid wenigstens eine anorganische Verbindung der Summenformel MO₃₋ₓ mit M=Mo und/oder W und 0≤x≤1 enthält. Mit anderen Worten kann es vorgesehen sein, dass zusätzlich zu dem oder den Mischoxid(en) wenigstens ein nicht als Mischkristall vorliegendes Molybdänoxid und/oder Wolframoxid verwendet wird, wobei das betreffende Molybdän- und/oder Wolframoxid MoO₃ und/oder WO₃, MoO₂ und/oder WO₂ und/oder ein gegenüber MoO₃ und/oder WO₃ sauerstoffdefizitäres Oxid ist, dessen Sauerstoffgehalt zwischen denjenigen von MoO₃/WO₃ und MoO₂/WO₂ liegt. Dementsprechend kann x beispielsweise Werte von 0, 0,01, 0,02, 0,03, 0,04, 0,05, 0,06, 0,07, 0,08, 0,09, 0,10, 0,11, 0,12, 0,13, 0,14, 0,15, 0,16, 0,17, 0,18, 0,19, 0,20, 0,21, 0,22, 0,23, 0,24, 0,25, 0,26, 0,27, 0,28, 0,29, 0,30, 0,31, 0,32, 0,33, 0,34, 0,35, 0,36, 0,37, 0,38, 0,39, 0,40, 0,41, 0,42, 0,43, 0,44, 0,45, 0,46, 0,47, 0,48, 0,49, 0,50, 0,51, 0,52, 0,53, 0,54, 0,55, 0,56, 0,57, 0,58, 0,59, 0,60, 0,61, 0,62, 0,63, 0,64, 0,65, 0,66, 0,67, 0,68, 0,69, 0,70, 0,71, 0,72, 0,73, 0,74, 0,75, 0,76, 0,77, 0,78, 0,79, 0,80, 0,81, 0,82, 0,83, 0,84, 0,85, 0,86, 0,87, 0,88, 0,89, 0,90, 0,91, 0,92, 0,93, 0,94, 0,95, 0,96, 0,97, 0,98, 0,99 oder 1,00 sowie sämtliche möglichen Zwischenwerte annehmen. Mit Hilfe dieser Verbindungen, einzeln oder in beliebiger Kombination, werden auf besonders einfache und kostengünstige Weise ein möglichst niedriger Oberflächen-pH-Wert und damit eine besonders gute antimikrobielle Wirksamkeit erzielt, da sich alle genannten Verbindungen bei Kontakt mit Wasser in Molybdän- bzw. Wolframsäure und/oder höhere saure Oligomolybdate bzw. Oligowolframate umwandeln. Darüber hinaus weisen insbesondere die sauerstoffdefizitären Verbindungen aufgrund der unterschiedlichen Oxidationsstufen von Mo/W, das heißt im Wesentlichen +IV, +V und/oder +VI, zusätzlich ein vergleichsweise hohes Oxidationspotential, magnetische Eigenschaften und/oder elektrische Leitfähigkeit auf, wodurch zusätzliche antimikrobielle Effekte erzielt werden können.

Besonders einfach und kostengünstig können die Verbindungen der allgemeinen Formel MO₃₋ₓ durch partielle Oxidation von M und/oder MO₂ und/oder durch partielle Reduktion von MO₃ hergestellt werden. Mit anderen Worten können die Metalle Mo/W und/oder deren Dioxide MoO₂/WO₂ partiell aufoxidiert werden, um zu den Verbindungen der allgemeinen Formel MO₃₋ₓ mit x<1 zu gelangen. Alternativ oder zusätzlich können die jeweiligen Trioxide MoO₃ und/oder WO₃ als Edukte verwendet und partiell reduziert werden, um zu den Verbindungen der allgemeinen Formel MO₃₋ₓ mit 0<x<1 zu gelangen.

Die Verbindungen der vorstehend genannten allgemeinen Formel MO₃₋ₓ mit 0≤x≤1 können beispielsweise mittels eines Wirbelschichtreaktors und/oder durch chemische Gasphasenabscheidung und/oder durch physikalische Gasphasenabscheidung und/oder durch Sputtern und/oder plasmaassistiert und/oder in einer kontrollierten Atmosphäre hergestellt werden. Die Verwendung eines Wirbelschichtreaktors bietet verschiedene Vorteile. Einerseits können die vorzugsweise partikelförmigen Edukte kontrolliert mit einem Fluid in einen fluidisierten Zustand versetzt werden, wobei als Fluid beispielsweise ein Reaktionsgas oder -gasgemisch verwendet werden kann, mittels welchem die partielle Oxidation und/oder Reduktion durchführbar ist. Alternativ oder zusätzlich kann über das Fluid eine gewünschte Reaktionstemperatur eingestellt werden. Ebenso können die fluidisierten Edukte durch eine entsprechende Fluidströmung, beispielsweise ringförmig, an einer Energiequelle, beispielsweise einer Flamme und/oder einer Plasmaquelle, vorbeigeführt werden, wodurch die Kontaktzeit für die einzelnen Partikel und damit ihr Oxidations- bzw. Reduktionsgrad besonders präzise eingestellt werden können. Ebenso kann der Eduktstrom gezielt mit einem Reaktand bzw. Reaktandengemisch versetzt oder an einem Reaktandenstrom vorbeigeleitet werden. Weiterhin können mit Hilfe des Wirbelschichtreaktors zusätzlich zu der Oxidation bzw. Reduktion weitere Funktionalisierungen der anorganischen Molybdän- und/oder Wolfram-Verbindungen vorgenommen werden. Beispielsweise können die Molybdän- und/oder Wolfram-Verbindungen vor, während und/oder nach der Oxidation bzw. Reduktion mit einer Funktionsschicht, beispielsweise einer Reaktandenschicht, einer Hydrophilierungsschicht und Ähnlichem versehen werden. Mit Hilfe von chemischer und/oder physikalischer Gasphasenabscheidung kann der erfindungsgemäße Verbundwerkstoff vorteilhafterweise unmittelbar hergestellt werden, indem der wenigstens eine weitere Werkstoff direkt mit der Molybdän- und/oder Wolfram-haltigen Verbindung beschichtet wird. Entsprechende Vorteile ergeben sich bei einem Schichtauftrag mittels Sputtern. Weiterhin kann vorgesehen sein, dass die Reaktion durch Einstellen einer kontrollierten Atmosphäre gesteuert wird. Beispielsweise kann eine Reduktion in einer kontrollierten Wasserstoffatmosphäre stattfinden, während zur Einstellung oxidativer Bedingungen beispielsweise Sauerstoff, Ozon, Wasserstoffperoxid, Chlor oder andere oxidative Verbindungen, einzeln und in beliebiger Kombination verwendet werden können. Grundsätzlich ist jedoch auch eine nasschemische Herstellung möglich.
Der Verbundwerkstoff gemäß dem zweiten Erfindungsaspekt und/oder das wenigstens eine dotierte Mischoxid eignet sich zur Herstellung eines Gegenstands mit einer antimikrobiell wirksamen Oberfläche, wobei das Mischoxid die Summenformel MoₓW₁₋ₓM_{y}O_{z} besitzt, in welcher 0<x<1, 0,01 ≤y≤2 und 2,0≤z≤3,0 betragen und M ein von Mo und W verschiedenes Metall-Ion bezeichnet, wobei zum Dotieren Fremdatome M ausgewählt aus der Gruppe Na, Cu, Bi, V, Ti und Zn in das Mischoxid eingebracht werden. Der Gegenstand kann aus einer Gruppe ausgewählt sein, die Haushaltswaren, Konsumgüter, industrielle Geräte und Komponenten, Schiffsanstriche, Fassadenanstriche, Tanks, Kabel, Beschichtungen, Leitungen, Rohre, Komponenten der Erdölexploration, Erdölförderung und Erdöllagerung, Medizintechnik, Lebensmitteltechnik, Sanitäranlagen, Verpackungen, Textilien, Möbel, Gebäudeelemente, Einrichtungsgegenstände, Klinken, Schalter, Sitze, Tastaturen und Ausstattungen von Kliniken, Arztpraxen, Pflegeeinrichtungen, Kindertagesstätten, Schulen und öffentlich zugänglichen Gebäuden umfasst. Das Mischoxid ist mittels eines Verfahrens gemäß dem ersten Erfindungsaspekt hergestellt. Der Gegenstand bzw. das Produkt kann weiterhin beispielsweise als Implantat, Katheter, Stent, Knochenimplantat, Zahnimplantat, Gefäßprothese, Endoprothese, Exoprothese, Kabel, Schlauch, Lebensmittelverpackung, Behälter, Kraftstofftank, Haushaltsprodukt, Schalter, Armatur, Tastatur, Maus, Joystick, Gehäuse, Textil, Faden, Bekleidungsstück, Möbel- und/oder Innenausbauteil, Haushaltsgerät, Kreditkarte, Handygehäuse, Münze, Geldschein, Türschnalle, Kühlschrank, Rieselkörper im Kühlturm, Lackbeschichtung, Fliese oder ein Teil der Innenausstattung eines Gebäudes oder öffentlichen Verkehrsmittels ausgebildet sein. Weiterhin kann vorgesehen sein, dass der Gegenstand als Lager- und Transportbehälter oder als Leitung für Kohlenwasserstoffe, Treibstoffe, Lösungsmittel und organische Flüssigkeiten ausgebildet ist. Ebenso eignet sich das Verbundmaterial zur Herstellung von Gegenständen und Produkten, die im häufigen Berührungskontakt mit Lebewesen stehen. Eine weitere vorteilhafte Verwendung liegt in Bauteilen für Klimaanlagen. Die Kühlrippen, die üblicherweise aus einer Cu- oder AI-Legierung bestehen, können in vorteilhafter Weise mit dem erfindungsgemäßen Verbundwerkstoff beschichtet oder aus diesem hergestellt werden. Auch die Schächte von Klimaanlagen in Gebäuden können antimikrobiell ausgeführt werden, indem der Verbundwerkstoff dem Schachtmaterial zugesetzt wird, das Schachtmaterial mit diesen beschichtet wird oder wenn das Schachtmaterial aus dem Verbundwerkstoff besteht. Auch Luftbefeuchter können mit entsprechenden antimikrobiellen Eigenschaften versehen werden. Außerdem kann der Verbundwerkstoff in Kabeln und/oder zur Herstellung von Kabeln verwendet werden.

Der Verbundwerkstoff kann als Beschichtungsmittel, insbesondere als Anstrichmittel, Lack und/oder Antifouling-Anstrich, ausgebildet sein. Unter einem Anstrichmittel werden Ausführungsformen des Verbundwerkstoffs verstanden, die eine flüssige bis pastenförmige Konsistenz besitzen und die auf Oberflächen aufgetragen einen physikalisch oder chemisch trocknenden Anstrich ergeben. Hierdurch können die vorteilhaften Eigenschaften des erfindungsgemäßen Verbundwerkstoffs besonders flexibel für beliebige Gegenstände und Oberflächen verwirklicht werden. Wichtige Ausgestaltungen sind beispielsweise Anti-Fouling Anstriche, z. B. für Schiffe, sowie antimikrobielle Ausstattung im Gesundheitswesen, der Industrie, dem Lebensmittelbereich und dem privaten Sektor.

Weitere Merkmale der Erfindung ergeben sich aus den Ansprüchen, den Ausführungsbeispielen sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen. Dabei zeigt:
- Fig. 1: ein schematisches Flussdiagramm eines Verfahrens zum Herstellen eines Mischoxids der Summenformel MoₓW₁₋ₓM_{y}O_{z}, in welcher 0<x<1, 0,01≤y≤2 und 2,0≤z≤3,0 betragen und M ein von Mo und W verschiedenes Metall-Ion bezeichnet;
- Fig. 2: eine Prinzipdarstellung einer Sprühtrocknungsvorrichtung;
- Fig. 3: eine Fotographie einer Glasplatte mit einem kontinuierlichen Gradienten von Mischoxiden der Formel MoₓW₁₋ₓO₃, wobei auf der linken Seite x=1 (MoO₃) und auf der rechten Seite x=0 (WO₃) ist; und
- Fig. 4 bis 6: Fotographien mehrerer Blutagarplatten aus Versuchreihen zur Untersuchung der antimikrobiellen Wirksamkeit mehrerer erfindungsgemäßer Verbundwerkstoffe.

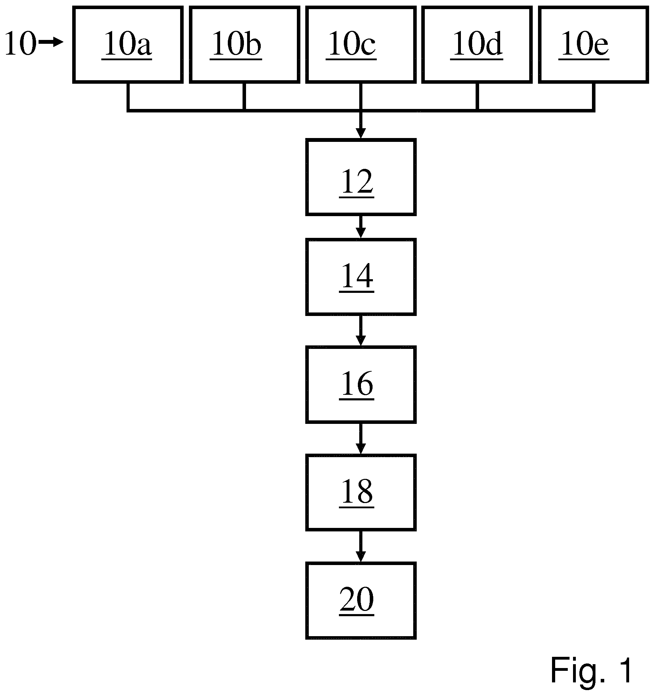

Fig. 1 zeigt ein schematisches Flussdiagramm eines Verfahrens zum Herstellen dotierter und/oder undotierter Mischoxide der Summenformel MoₓW₁₋ₓM_{y}O_{z}, in welcher 0<x<1, 0,01 ≤y≤2 und 2,0≤z≤3,0 betragen und M ein von Mo und W verschiedenes Metall-Ion aus der Gruppe Na, Cu, Bi, V, Ti und Zn bezeichnet. Die Herstellung dieser Mischoxide erfolgt in mehreren Teilschritten. Es können nicht-erfindungsgemäße "reine" Mischkristalle, die nur Mo, W, O und gegebenenfalls Leerstellen im Kristallgitter enthalten, hergestellt werden. Es ist aber auch möglich, die Mischoxide erfindungsgemäß gezielt mit Metallen bzw. Metall-Ionen wie beispielsweise Cu, Bi, V und Zn zu versetzen bzw. zu dotieren. Diese Metalle werden aufgrund ihrer von Mo und W unterschiedlichen Atomradien nicht direkt in das Kristallgitter eingebaut, sondern liegen als Molybdate und Wolframate, teilweise auch gemischt, bzw. als Oxide im Mischoxid vor.

In einem ersten Schritt 10 werden zunächst geeignete Molybdänverbindungen 10a und Wolframverbindungen 10b und weitere Edukte wie beispielsweise Zink-Verbindungen 10c, Bismut-Verbindungen 10d und/oder Kupfer-Verbindungen 10e bereitgestellt. Die Molybdänverbindungen 10a können beispielsweise ADM, APM, MoO₂, MoO₃, MoO₃*x H₂O, POM (POM = Polyoxometallat) und/oder metallisches Molybdän umfassen. Die Wolframverbindungen 10b können beispielsweise AMT, APT, WO₂, WO₃, WO₃*x H₂O, POM und/oder metallisches Wolfram umfassen. Ebenso können grundsätzlich verschiedene weitere Molybdän- und Wolframverbindungen wie beispielsweise deren Karbide, Nitride, Silizide und Sulfide verwendet werden, da diese Verbindungen ebenfalls in Oxide umgewandelt werden können. Die Zink-Verbindungen 10c können beispielsweise ZnO, Salze wie Zn(NO₃)₂ oder metallisches Zink umfassen. Die Bismut-Verbindungen 10d können beispielsweise Bi₂O₃, Salze wie Bi(NO₃)₃ oder metallisches Bismut umfassen. Die Kupfer-Verbindungen 10e können beispielsweise CuO, Salze wie Cu(NO₃)₂ oder metallisches Kupfer umfassen. Für Zn, Bi, Cu und V sind Salze besonders vorteilhaft. Nitratsalze sind in der Regel am besten geeignet, weil kein störendes Anion (z.B. Cl⁻, SO₄²⁻) bei einem gegebenenfalls anschließenden Kalzinierungsschritt zurückbleibt. Es ist aber auch möglich, reine Metalle, Legierungen oder deren Oxide zu verwenden, vorteilhaft als feines Pulver.

In einem Schritt 12 wird ein flüssiges Medium bzw. werden mehrere gleiche oder unterschiedliche flüssige Medien bereitgestellt, um eine oder mehrere Lösungen und/oder Suspensionen der bereitgestellten Edukte herzustellen. Als flüssige Medien eignen sich beispielsweise polare Lösungsmittel wie Wasser, Acetonitril oder Alkohole. Vor allem Methylalkohole haben sich als vorteilhaft herausgestellt. Kohlenwasserstoffe sind ebenfalls grundsätzlich geeignet. Der Feststoffgehalt in den Lösung(en)/Suspension(en) bzw. der Gehalt an gelöstem/suspendiertem Material kann in jeder Lösung/Suspension zwischen 0,1% und 90% betragen (nach Masse).

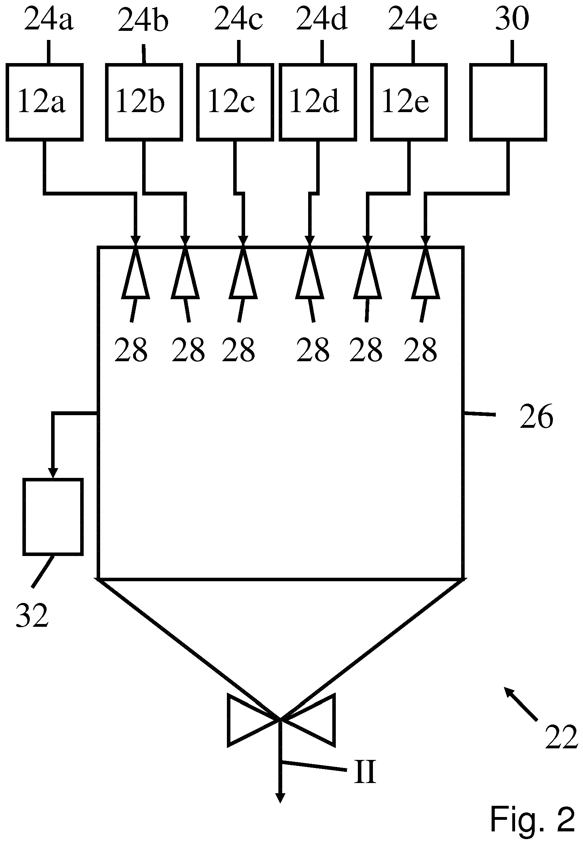

In einem Schritt 14 werden die Lösungen/Suspensionen einzeln oder gemeinsam verdüst (s. Fig. 2). Manche Ausgangsstoffe bilden beim Zusammenschütten der Lösungen/Suspensionen oder beim gemeinsamen Ansetzen einer Lösung/Suspension eine Trübung, beispielsweise Zinknitrat und ADM. In solchen Fällen ist ein separates Verdüsen dieser Stoffe besser.

Die Bildung der Mischoxide kann nun nach unterschiedlichen Verfahren erfolgen. Sprühtrocknung ist besonders vorteilhaft, da dieses Verfahren apparativ einfach und mit geringen Produktionskosten verbunden ist. Ebenfalls bewährt hat sich Gefriertrocknung. Auch möglich sind die Verfahren Verbrennungssynthese, Flammenhydrolyse, Gasphasensynthese, und Spraypyrolyse. Flammenbasierte Verfahren haben den Vorteil, besonders feine Teilchen zu erzeugen, deren mittlere Korngrößen im Nanometer-Bereich liegen können. Je nach Verfahren und Verfahrensführung fällt das Mischoxid bzw. fallen die Mischoxide in unterschiedlicher Korngrößenverteilungen und Feuchtigkeiten an.

In einem grundsätzlich optionalen Schritt 16 wird das in Schritt 14 hergestellte Pulver kalziniert. Darunter ist eine Wärmebehandlung bei 150 bis 1000°C zu verstehen. Sie kann beispielsweise in einem Festbett oder in einer Wirbelschicht erfolgen. Die Verweilzeiten der hergestellten Pulver liegen im Bereich von wenigen Sekunden bis zu mehreren Stunden. Es können oxidierende oder reduzierende Bedingungen sowie Schutzgas verwendet werden. Als Schutzgas haben sich CO₂, Argon oder Stickstoff bewährt. Für oxidierende Bedingungen können beispielsweise Luft, Sauerstoff, Ozon, Stickstoffdioxid oder Wasserstoffperoxid verwendet werden. Für reduzierende Bedingungen sind unter anderem Kohlenmonoxid und Wasserstoff gut geeignet. Das Kalzinieren hat einen wichtigen Einfluss auf die spezifische Oberfläche der Pulver sowie die darin enthaltenden Fehlstellen. Je höher die Kalzinierungstemperatur, desto geringer wird die spezifische Oberfläche des Mischoxids. Fehlstellen werden teilweise abgebaut, aber auch neu geschaffen. Besonders vorteilhaft ist eine Kalzinierungstemperatur im Bereich von 200 bis 400°C bzw. ene Verweilzeit von 0,5 bis 4 Stunden.

In einem grundsätzlich optionalen Schritt 18 wird das Pulver aus Schritt 14 und/oder Schritt 16 zerkleinert. Grundsätzlich kann die Reihenfolge der Schritte 16 und 18 vertauscht werden. Ebenso ist es möglich, die Schritte 16 und/oder 18 mehrfach bzw. in beliebiger Reihenfolge durchzuführen. Zum Zerkleinern sind unterschiedliche Verfahren möglich. Trockenmahlung wird bevorzugt, insbesondere mittels einer Kugelmühle oder über Strahlvermahlen (jet milling). Eine Korngröße von 0,1 bis 150 µm, gemessen über Laserbeugung/Laserstreuung, hat sich als antimikrobiell besonders wirksam gezeigt. Besonders vorteilhaft sind Korngrößen von höchstens 5 µm.

In einem Schritt 20 wird das Mischoxid in Werkstoffe, die antimikrobiell ausgestattet werden sollen, eingebracht oder auf deren Oberfläche aufgebracht, um einen Verbundwerkstoff zu erhalten. Vor allem Kunststoffe, Farben, Lacke und Keramiken lassen sich mit den Mischoxiden besonders einfach antimikrobiell ausstatten. Typischerweise werden bezogen auf die Gesamtmasse des Verbundwerkstoffs 0,5 bis 5% (Masse) der Mischoxide in die Werkstoffe eingebracht und/oder auf die Werkstoffe aufgebracht. Geeignete Verfahren sind beispielsweise Eincompoundieren für Kunststoffe (Extruder) und Einschneiden für Farben und Lacke (Dissolver). Für das direkte Aufbringen auf der Oberfläche sind unter anderem CVD, Schlickerguss und Sol-Gel Verfahren geeignet. Es ist möglich bezogen auf die Gesamtmasse des Verbundwerkstoffs 0,1 bis 80% (Masse) der Mischoxide in einen oder mehrere Werkstoffe einzubringen, entweder direkt als Endprodukt oder als Konzentrat für die Weiterverarbeitung (sog. Masterbatch).

Fig. 2 zeigt eine Prinzipdarstellung einer Sprühtrocknungsvorrichtung 22. Die Sprühtrocknungsvorrichtung 22 weist in Anzahl und Anordnung beispielhaft 5 Behälter 24, die zur besseren Unterscheidbarkeit mit den Bezugszeichen 24a-e bezeichnet sind, auf, in welchen die in Schritt 12 hergestellten Lösungen/Suspensionen 12a-12e aufgenommen sind. 12a bezeichnet dabei die Lösung/Suspension der Molybdänverbindung(en) 10a, 12b bezeichnet die Lösung/Suspension der Wolframverbindung(en) 10b, 12c bezeichnet die Lösung/Suspension der Zink-Verbindungen 10c, 12d bezeichnet die Lösung/Suspension der Bismut-Verbindungen 10d und 12e bezeichnet die Lösung/Suspension Kupfer-Verbindungen 10e. Optional mögliche Vanadium- und Titan-Verbindungen sind in dieser Grafik nicht dargestellt, können jedoch zusätzlich oder alternativ zu den anderen genannten Verbindungen eingesetzt werden. Die einzelnen Lösungen/Suspensionen 12a-12e können unabhängig voneinander in einen Trocknungsraum 26 der Sprühtrocknungsvorrichtung 22 eingebracht werden. Hierzu sind die Behälter 24a-e fluidisch mit entsprechenden Düsen 28 gekoppelt. Neben der Zuführung der einzelnen Lösungen/Suspensionen über einzelne Leitungen und Düsen 28 können diese vorher vereinigt und über eine gemeinsame Leitung und Düse 28 in die Sprühtrocknungskammer eingeleitet werden. Ferner ist es möglich, die Mischung der Lösungen direkt in einer speziellen Mehrstoffdüse (nicht gezeigt) unmittelbar vor der Verdüsung vorzunehmen. Über eine grundsätzlich optionale Zuführung 30 können weitere Verbindungen, insbesondere Gase oder Gasmischungen in den Trocknungsraum 26 eingebracht werden, beispielsweise um oxidierende oder reduzierende Reaktionsbedingungen zu schaffen. Ebenso können Schutzgase eingeleitet werden. Das flüssige Medium bzw. die flüssigen Medien werden während des Prozesses über die Unterdruckeinrichtung 32 aus dem Trocknungsraum 26 entfernt. Das oder die getrockneten Produkte fallen schwerkraftbedingt nach unten und können gemäß Pfeil II über entsprechende Entnahmesysteme aus dem Trocknungsraum 26 entfernt werden. Die Sprühtrocknungsvorrichtung 22 kann auf Wunsch mit einer Zerkleinerungseinrichtung (nicht gezeigt) und/oder einer Kalzinierungseinrichtung (nicht gezeigt) kombiniert werden.

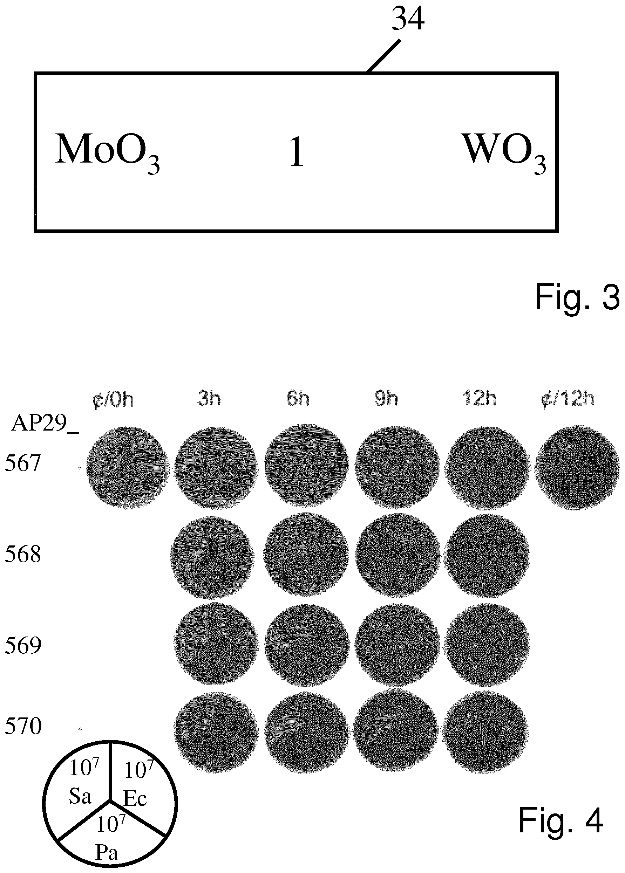

Fig. 3 zeigt eine Fotographie einer Glasplatte 34 mit einem kontinuierlichen Gradienten von nicht-erfindungsgemäßen Mischoxiden der Formel MoₓW₁₋ₓO₃. Die Herstellung erfolgte hier über PVD. Auf der linken Seite der Glasplatte 34 liegt reines MoO₃ (x=1) und auf der rechten Seite der Glasplatte 34 reines WO₃ (x=0) vor. In der Mitte der Glasplatte 34 ist x=0,5, so dass ein Mischoxid der Summenformel Mo_{0,5}W_{0,5}O₃ vorliegt. Auf diese Weise ist es besonders einfach möglich, ein Screening nach den wirksamsten Mischungsverhältnissen durchzuführen.

Auch die Metalle Kupfer, Vanadium, Bismut, Titan und Zink können als Molybdate/Wolframate, Oxide und/oder Bronzen vorliegen. Bei Bronzen handelt es sich um Einlagerungsverbindungen (Interkalationsverbindungen). Sie haben nur den Namen gemein mit der klassischen "Bronze", eine Cu-Sn-Legierung. Der Name Bronze steht bei Molybdän und Wolfram für eine Vielzahl von Verbindungen, konkret Wolframbronzen MₓWO₃ (0<x<=1) und Molybdänbronzen MₓMoO₃ (0<x<=1) [M = einwertiges aber auch höherwertiges Metall aber auch nicht-erfindungsgemäßes NH₄⁺). Der Name "Bronzen" wurde gewählt, weil sich die Farbe dieser Verbindungen, abhängig von x, über einen großen Bereich verändert. Darunter sind ganz herrliche Farbtönungen. Das Verhältnis (W+Mo) : O liegt bei etwa 1 : 3. Es existieren kubische, tetragonale und hexagonale Bronzen. Die realisierbare Grenzzusammensetzung wird von der Größe des Kations und dem Strukturtyp bestimmt. Ein Beispiel ist das kubische, nicht-erfindungsgemäße NaₓWO₃. in diesem Fall kann x den Wert 1 erreichen.

Im Folgenden werden 6 Substanzklassen an Mischoxiden, wobei der Begriff "Mischoxid" synonym mit "Mischkristall" verwendet wird, auch wenn es sich um amorphes oder teilamorphes Material handelt, näher diskutiert. Die 6 Substanzklassen sind in untenstehender Tabelle 1 ausgeführt. Der jeweilige Gehalt der einzelnen Komponenten ist in Mol-% angegeben, wohlwissend, dass für praktisches Hantieren (Einwiegen, Mischen, etc.) Massenprozente komfortabler sind.

**Tabelle 1: Zusammensetzung der mit Zn/Bi/Cu dotierten MoₓW₁₋ₓO_{z}-Mischoxide (Angaben in Mol-% der Elemente)**

| Verbindung | MoₓW₁₋ₓM_{y}O_{z} mit M=Zn, Bi, Cu | MoₓW₁₋ₓM_{y}O_{z} mit M=Zn, Bi | MoₓW₁₋ₓM_{y}O_{z} mit M=Zn | MoₓW₁₋ₓM_{y}O_{z} mit M=Bi | MoₓW₁₋ₓM_{y}O_{z} mit M=Cu | MoₓW₁₋ₓO_{z} |
|---|---|---|---|---|---|---|
| Anteil W | (0,98-0,90)·(25-75) | | | | | 25-75 |
| Anteil Mo | (0,98-0,90)·(25-75) | | | | | 25-75 |
| Anteil Zn | 2-10 | 2-10 | 2-10 | | | |
| Anteil Bi | | | | 2-10 | | |
| Anteil Cu | | | | | 2-10 | |
| Enthaltene Verbindung | MoₓW₁₋ₓM_{y}O₃-Mischkristall dotiert mit Zn-, Bi- und Cu-Wolframaten/- Molybdaten | MoₓW₁₋ₓM_{y}O₃-Mischkristall dotiert mit Zn- und Bi-Wolframaten/- Molybdaten | MoₓW₁₋ₓM_{y}O₃-Mischkristall dotiert mit Zn-Wolframaten/- Molybdaten | MoₓW₁₋ₓM_{y}O₃-Mischkristall dotiert mit Bi-Wolframaten/- Molybdaten | MoₓW₁₋ₓM_{y}O₃-Mischkristall dotiert mit Cu-Wolframaten/- Molybdaten | MoₓW₁₋ₓM_{y}O₃-Mischkristall |

Die Möglichkeit, zusätzlich oder anstatt mit Zn, Bi oder Cu mit Vanadium (V) und/oder Titan (Ti) oder anderen Metallen (M) zu dotieren, ist in Tabelle 1 nicht dargestellt, jedoch ebenfalls möglich. Als besonders vorteilhaft haben sich W-Mo-Mischoxide gezeigt, die ein molares W:Mo - Verhältnis von 3:1, 1:1 und 1:3 aufwiesen. Es ist aber möglich, das Verhältnis von Mo:W bzw. von W:Mo im Bereich 1:250 zu variieren.

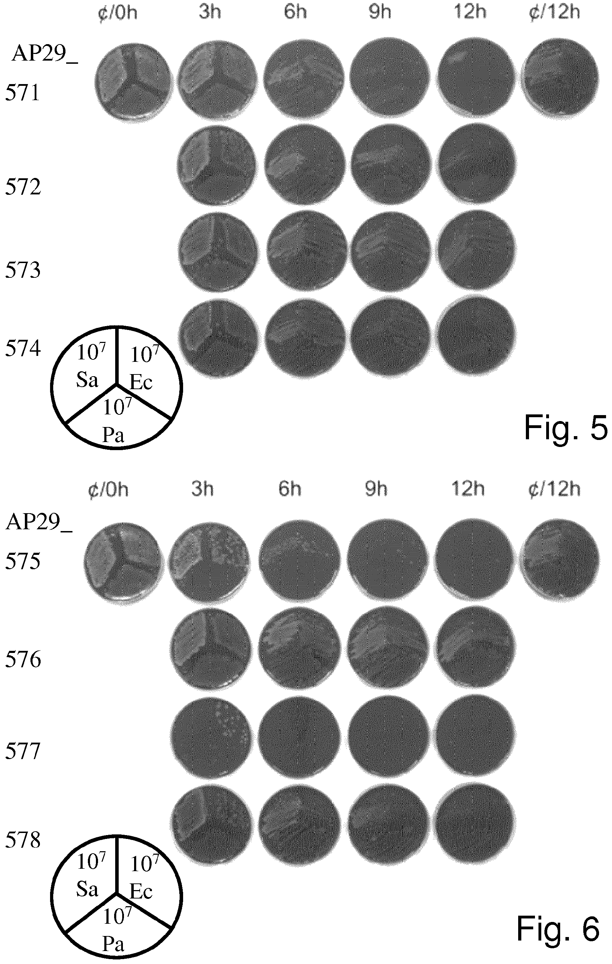

Die erfindungsgemäß hergestellten Verbindungen sowie die erfindungsgemäßen Verbundwerkstoffe weisen eine exzellente antimikrobielle Wirksamkeit auf. Fig. 4 bis Fig. 6 zeigen jeweils Fotographien mehrerer Blutagarschalen aus Versuchreihen zur Untersuchung der antimikrobiellen Wirksamkeit erfindungsgemäßer Verbundwerkstoffe. Die Blutagarschalen waren jeweils mit einer Blutagarplatte versehen, die in drei Sektoren mit jeweils unterschiedlichen Bakterientests unterteilt wurden. Die Versuche erfolgten nach der Auftropfmethode mit den drei Keimen E.c., S.a. und P.a. Bei der Auftropfmethode wird auf einen antimikrobiellen Probekörper jeweils ein 10⁵-10⁹ KBE/ml an Keimen enthaltender Tropfen von beispielsweise 100 µl aufgegeben. Alle 3 Stunden werden beispielsweise 10 µl aus dem Tropfen abgezogen und auf der gedrittelten Agarplatte verteilt und anschließend bei 37°C für 10-24 Stunden bebrütet. Je mehr Keime danach auf der Agarplatte zu sehen sind, desto mehr Keime existieren noch im Tropfen auf dem Probekörper. Neben dieser Auftropfmethode hat sich insbesondere die Methode der Ausrollkultur zur Untersuchung der antimikrobiellen Wirkung von Verbundmaterialien gut bewährt (hier nicht dargestellt). Die untersuchten Verbundmaterialien werden dabei zur Untersuchung ihrer antimikrobiellen Wirksamkeit in Form eines Zylinders in entsprechende Keimsuspensionen gegeben. Es kommt zum oberflächlichen Anwachsen von Keimen. Nach 3, 6, 9 und 12 Stunden werden die Proben über eine Agarplatte gerollt und dazwischen in eine sterile, physiologische Kochsalzlösung gegeben. Nach diesem Abrollvorgang werden die Agarplatten bzw. Petrischalen bei 37°C für 10-24 Stunden bebrütet, damit die vom Probekörper übertragenden Keime anwachsen und sichtbar gemacht werden können. Die Agarplatten werden fotografiert und bezüglich der keimreduzierenden bzw. keimtötenden Wirkung des betreffenden Verbundmaterials beurteilt. Diese wiederholte Abrolltätigkeit in 3-stündigem Abstand gibt Aufschluss, ob und mit welchem Effizienzgrad eine keimreduzierende oder keimtötende Wirkung eintritt. Die Ausrollmethode erfasst auch die Verminderung der Adhärenz von Keimen auf Oberflächen. Sie ist besonders gut geeignet, Kontaktbiozide zu beurteilen, wohingegen Verfahren wie Hemmhoftestung bevorzugt für Wirksysteme, die migrierende Stoffe enthalten bzw. Ionen oder organische Verbindungen freisetzen müssen wie Silber, geeignet sind. Der Wirkmechanismus der Mischoxide ist ähnlich wie bei MoO₃ und WO₃ und damit ein Phänomen direkt an der Oberfläche bzw. Grenzfläche des betreffenden Verbundwerkstoffs, wo der pH-Wert abgesenkt wird, während der pH-Wert einer darüberstehenden Lösung nicht merklich verändert wird. Neben der pH-Wert-Absenkung an der Oberfläche beruht die antimikrobielle Wirksamkeit der Mischoxide bzw. der Mischoxide in Werkstoffe eingebracht auf weiteren Effekten, darunter auf elektrostatischen Wechselwirkungen zwischen den Mischoxiden und den Zellwänden der Keime. Diese Wechselwirkungen sind in der Regel umso stärker, je größer die spezifische Oberfläche der Mischoxide und/oder die Dichte ihrer Leer- bzw. Fehlstellen ist. Die Ausrollmethode lässt sich für die Untersuchung verschiedener Mikroorganismen anwenden. Die Untersuchungen zum Wirkungsnachweis der erfindungsgemäßen Verbundmaterialien erfolgten getrennt für die Referenzstämme Staphylococcus aureus (S.a., Suspension mit 10⁷ KBE/ml (Koloniebildende Einheiten pro Milliliter)), Escherichia coli (E.c., Suspension mit 10⁷ KBE/ml) und Pseudomonas aeroginosa (P.a., Suspension mit 10⁷ KBE/ml).

Tabelle 2 gibt die Zusammensetzung und Herstellungsdetails der in Fig. 4 bis Fig. 6 geprüften Verbundwerkstoffe enthaltend nicht-erfindungsgemäß hergestellte Mischoxide der allgemeinen Formel MoₓW₁₋ₓO_{z} mit z=2,8 bis 3,0 wieder. TPU1180A bezeichnet dabei ein thermoplastisches Polyurethan, das beispielsweise unter dem Handelsnamen Elastollan 1180A bei BASF erhältlich ist. Die antimikrobielle Wirksamkeit der Verbindungen wurde jedoch in weiteren Kunststoffen wie PP, PE, PC, PS, ABS, PVC sowie Silikon, Pulverlack, Flüssiglacken, Epoxidharz, und anderen Werkstoffen in ähnlicher Ausprägung nachgewiesen. Vor allem in apolaren Werkstoffen konnte durch zusätzlich eingebrachte Hydrophilierungsmittel (Feuchthaltemittel) eine Steigerung der antimikrobiellen Wirksamkeit dargestellt werden. Daneben kann zusammen mit dem Mischoxid auch TiO₂ eingesetzt werden. In Fig. 4 bis Fig. 6 sind zusätzlich auch mit Ø gekennzeichnete Kontrollproben ohne antimikrobielle Ausstattung dargestellt. Diese Kontrollproben wurden zu Beginn und zum Ende der Testung angefertigt um zu zeigen, dass die Keime über die Dauer des Experiments nicht von alleine absterben.

| **Nr.** | **Zusammensetzung** | **Fig.** | **Molverhältnis Mo:W** | **Pulver** | **Kalziniert** |
|---|---|---|---|---|---|
| AP29_567 | TPU1180A + 2% Mischoxid | Fig. 4 | 1:1 | fein | 300°C |
| AP29_568 | TPU1180A + 2% Mischoxid | Fig. 4 | 1:1 | grob | 300°C |
| AP29_569 | TPU1180A + 2% Mischoxid | Fig. 4 | 1:1 | fein | 400°C |
| AP29_570 | TPU1180A + 2% Mischoxid | Fig. 4 | 1:1 | grob | 400°C |
| AP29_571 | TPU1180A + 2% Mischoxid | Fig. 5 | 1:3 | fein | 300°C |
| AP29_572 | TPU1180A + 2% Mischoxid | Fig. 5 | 1:3 | grob | 300°C |
| AP29_573 | TPU1180A + 2% Mischoxid | Fig. 5 | 1:3 | fein | 400°C |
| AP29_574 | TPU1180A + 2% Mischoxid | Fig. 5 | 1:3 | grob | 400°C |
| AP29_575 | TPU1180A + 2% Mischoxid | Fig. 6 | 3:1 | fein | 300°C |
| AP29_576 | TPU1180A + 2% Mischoxid | Fig. 6 | 3:1 | grob | 300°C |
| AP29_577 | TPU1180A + 2% Mischoxid | Fig. 6 | 3:1 | fein | 400°C |
| AP29_578 | TPU1180A + 2% Mischoxid | Fig. 6 | 3:1 | grob | 400°C |

Wie man in Fig. 4 bis Fig. 6 erkennt, wiesen alle Proben eine hervorragende antimikrobielle Wirksamkeit auf, so dass spätestens nach 12h eine weitgehende oder vollständige Keimabtötung eintrat. Für eine besonders hohe antimikrobielle Wirksamkeit haben sich in der Praxis Gewichtsanteile von 0,05 bis 15% (nach Masse) der erfindungsgemäß hergestellten Mischoxide in Kunststoffen, Farben, Lacken und Keramik als vorteilhaft gezeigt. Besonders vorteilhaft ist ein Konzentrationsbereich von 0,5 bis 5% nach Masse, insbesondere 1-3%.

Weitere in der oben genannten Weise auf ihre antimikrobielle Wirksamkeit getesteten und für besonders gut wirksam befundene, nicht-erfindungsgemäße Mischoxide wiesen die allgemeine Summenformel MoₓW₁₋ₓO_{z} mit 0,64≤x≤0,68 und 0,27≤z≤3,0 auf, beispielsweise Mo_{0,66}W_{0,34}O₃.

Bei Beschichtung von Oberflächen, z. B. mit Hilfe von physikalischen (PVD)- oder chemischen (CVD)-Gasabscheidungsverfahren, kann die Oberfläche des Verbundwerkstoffs bis zu 100% des Mischoxids enthalten. Von Kunststoffen, Farben und Lacken lassen sich auch Konzentrate (Masterbatches) mit 15% bis 95% (nach Masse) an Mischoxid in einem Träger herstellen, welche später zur Herstellung des eigentlichen Produkts wieder verdünnt werden. Folgende Materialien können beispielsweise antimikrobiell ausgestattet werden, indem die hier beschriebenen Mischoxide zugesetzt werden: Polymere und Kunststoffe, darunter die Therrmoplaste PE, PP, PC, ABS, PS, PU, PVC, PET und POM, Elastomere, Duroplaste, die Stoffe TPU, TPE, TPV, Polymilchsäure, Silikon, Pulverlacke, Flüssiglacke, Keramik, Melamin, Methylacrylat, Holz, Epoxidharz, Wachse, Emulsionen und flüssige und feste Reinigungs- und Desinfektionsmittel. Bei letzteren kann durch den Zusatz der Mischoxide eine Remanenz (Steigerung der Wirkdauer) sowie eine Erhöhung der Wirksamkeit erzielt werden. Zur weiteren Steigerung der antimikrobiellen Wirksamkeit kann der auszustattende Verbundwerkstoff zusätzlich hydrophiliert werden. Dies ist beispielsweise durch Zusatz eines Feuchthaltemittels, Antistatikums, Anti-Fog Agents und/oder eines Tensids möglich.

Die erfindungsgemäßen Mischoxide der Summenformel MoₓW₁₋ₓM_{y}O_{z}, in welcher 0<x<1, 0,01 ≤y≤2 und 2,0≤z≤3,0 betragen und M ein von Mo und W verschiedenes Metall-Ion aus der Gruppe Na, Cu, Bi, V, Ti und Zn bezeichnet, weisen zusammenfassend gegenüber reinem MoO₃ und reinem WO₃, gegenüber Mischungen aus reinem MoO₃ und reinem WO₃ sowie gegenüber gängigen antimikrobiellen Wirkstoffen folgende Vorteile im Hinblick auf die antimikrobielle Ausstattung von Verbundwerkstoffen auf:
- Sehr geringe Löslichkeit in Wasser, Alkoholen und anderen Lösungsmitteln
- Sehr geringes toxikologisches Potential, keine Hinweise auf Karzinogenität
- Geringe Eigenfärbung, gut überfärbbar
- Kostenersparnis, da mit weniger Dosierung als bei MoO₃ und WO₃ der gleiche antimikrobielle Effekt erzielt werden kann.
- Breiteres Wirkungsspektrum

Folgende, bereits im System reines MoO₃ und reines WO₃ bzw. bei makroskopischen Mischungen aus MoO₃ und WO₃ beobachteten positiven Effekte zur antimikrobiellen Ausstattung zeigen sich auch bei den erfindungsgemäßen Mischoxiden:
- Robustheit im Praxiseinsatz: Keine Inaktivierung durch schwefelhaltige Verbindungen, Eiweiß, Proteine und Schweiß
- Langanhaltende, starke Wirksamkeit gegen ein breites Spektrum an Keimen: Bakterien, Viren, Pilze und Algen.
- Auch wirksam gegen Antibiotika-resistente Keime, darunter multiresistente Keime wie MRSA, ESBL, VRE und Legionellen

Ohne auf die folgenden Theorien festgelegt werden zu wollen, gehen die Erfinder davon aus, dass die antimikrobielle Wirksamkeit der neuen Mischoxide auf mehreren Effekten beruht, teilweise in unterschiedlich starker Ausprägung, die hier aufgezählt sind:
- Bildung von sauren Zentren bzw. sauren Oberflächen bei Kontakt mit Wasser
- Destabilisierung der Zellwände von Keimen durch elektrostatische Wechselwirkungen (Zeta-Potential, Dichte an Fehlstellen bzw. Ladungen/freien Valenzen an der Oberfläche)
- Oligodynamischer Effekt

Vor allem in Anwesenheit von Zink ist zudem ein Photoeffekt beobachtet worden.

In einem weiteren Ausführungsbeispiel werden zusätzlich zu dem oder den Mischoxid(en) die Molybdate/Wolframate CaMO₄, ZnMO₄, BiMO₄, VMO₄, CuMO₄ und Ag₂MO₄ mit M=Mo, W, einzeln oder in beliebiger Kombination, verwendet und in einen Verbundwerkstoff eingearbeitet. Die Herstellung dieser Molybdate/Wolframate erfolgt, indem MoO₃ und/oder WO₃ mit den entsprechenden Carbonaten innig vermischt und auf Temperaturen von etwa 400 °C bis 800°C erhitzt werden. Dabei können grundsätzlich auch unterschiedliche Carbonate verwendet werden, um entsprechende Mischmolybdate bzw. Mischwolframate zu erhalten. Die Reaktion kann durch Entfernen des entstehenden CO₂ aus dem Reaktionsgemisch in Richtung der gewünschten Produkte getrieben werden. Sobald kein Carbonat mehr nachweisbar ist, ist die Umsetzung vollständig erfolgt. Die genannten Verbindungen zeigen neben einer guten antimikrobiellen Wirkung auch eine besonders hohe Licht- und UV-Stabilität. Die Prüfung der UV-Beständigkeit kann in Anlehnung an DIN EN 438-2, Abschnitt 27 durchgeführt werden. Hierbei wird eine Probe eines Verbundwerkstoffs hergestellt, indem eine oder mehrere der genannten Molybdate bzw. Wolframate mit einem oder mehreren Mischoxiden und wenigstens einem weiteren Werkstoff zu einem Verbundwerkstoff verbunden werden. Die Molybdate bzw. Wolframate sowie die Mischoxide können dabei als Schicht bzw. Bestandteil einer Schicht vorliegen und/oder im wenigstens einen weiteren Werkstoff verteilt vorliegen. Der Verbundwerkstoff wird für 60 Minuten einer Bestrahlung ausgesetzt. Gleiches erfolgt mit einer analog, aber ohne Zusatz der genannten Molybdän-/Wolfram-Verbindungen hergestellten Vergleichsprobe ("Standard-Produkt"). Die Auswertung erfolgt dann anhand eines visuellen Vergleichs der antimikrobiell ausgerüsteten und der nicht ausgerüsteten Probe und wird beispielsweise wie folgt bewertet:
1: kein wahrnehmbarer Unterschied zum Standard-Produkt
2: schwer wahrnehmbarer Unterschied zum Standard-Produkt
3: eindeutig wahrnehmbarer Unterschied zum Standard-Produkt
4: gerade noch akzeptabler Unterschied zum Standard-Produkt
5: nicht akzeptabler Unterschied zum Standard-Produkt

Für Verbundwerkstoffe, die neben Mischoxiden auch Molybdate/Wolframate enthalten, werden dabei stets Werte von 1 oder höchstens 2 erhalten.

In einem weiteren nicht-erfindungsgemäßen Ausführungsbeispiel enthält der Verbundwerkstoff neben 2 Gew.-% Mischoxid(en) zusätzlich zwischen 0,1 Gew.-% und 2 Gew.-% Verbindungen der allgemeinen Formel MO₃₋ₓ mit 0<x<1 und M=W, Mo. Hierzu wurden MoO₂ und WO₂, einzeln oder in bestimmten Mischungsverhältnissen, partiell oxidiert. Die resultierenden Oxide bzw. Mischoxide wiesen ebenfalls hervorragende antimikrobielle Eigenschaften auf, wenn sie zusammen mit Mischoxid(en) im Verbundwerkstoff vorlagen.

Als Werkstoffe bzw. Matrix für die Herstellung des Verbundwerkstoffs kommen grundsätzlich beispielsweise thermoplastische oder duroplastische Kunststoffe, Farben, Lacke, Silikone, Gummi, Kautschuk, Melamin, Acrylate, Methylacrylate, Wachse, Epoxydharze, Glas, Metall, Keramik und weitere in Frage. Der Werkstoff, in welchen zum Zwecke der antimikrobiellen Ausstattung das oder die Molybdän- bzw. Wolfram-Verbindung(en) eingebracht wird bzw. werden, kann eine feste und/oder flüssige Matrix bilden. Es kann vorgesehen sein, dass die Molybdän- bzw. Wolfram-Verbindungen derart zugegeben werden, dass sie zwischen 0,1 % und 10 % (Gewichts- oder Volumenprozente) des Gesamtgewichts bzw. Gesamtvolumens ausmachen. Weiterhin kann vorgesehen sein, dass die Molybdän- bzw. Wolfram-Verbindungen in partikulärer Form mit mittleren Teilchengrößen zwischen 0,1 µm und 100 µm verwendet werden.

Beispielsweise kann der wenigstens eine weitere Werkstoff hydrophobe Polymere wie Silikone, Polypropylen (PP), Acrylnitril-Butadien-Styrol (ABS), Polycarbonat (PC) oder Polystyrol (PS) umfassen bzw. aus diesen bestehen. Ebenso vorgesehen sein können Phenol-Harze, Phenolformaldehyd-Harze, Melamin-Harze, Melaminformaldehyd-Harze, Harnstoff-Harze, Harnstoffformaldehyd-Harze und polymeres Diphenylmethandiisocyanat sowie beliebige Mischungen daraus. Weiterhin kann der Verbundwerkstoff als weiteren Werkstoff Polyethylen (PE), Polyethylenterephthalat (PET), Polyvinylchlorid (PVC), Polystyrol (PS), Polycarbonat (PC) oder ein Poly(meth)acrylat (z. B. PAA, PAN, PMA, PBA, ANBA, ANMA, PMMA, AMMA, MABS und/oder MBS) umfassen. Die Verwendung von thermoplastischen Elastomeren erlaubt die Herstellung von Oberflächen mit gummielastischen Eigenschaften, in welchen die wenigstens eine Molybdän-/Wolfram-haltige Verbindung aufgenommen bzw. gehalten ist. Das oder die thermoplastischen Elastomere können beispielsweise den Klassen TPO, TPV, TPU, TPC, TPS oder TPA bzw. beliebigen Mischungen hieraus angehören, wobei sich insbesondere thermoplastische Elastomere auf Urethanbasis (TPUs) als vorteilhaft gezeigt haben. Die Verwendung eines Reaktivlacks erlaubt die Herstellung von mechanisch besonders widerstandsfähigen Oberflächen, wobei der Reaktivlack durch chemische Reaktion vorzugsweise bereits bei Raumtemperatur erhärtet. Der Reaktivlack kann grundsätzlich als Ein- oder Mehrkomponentenlack vorliegen bzw. verwendet werden. Ebenso kann der Verbundwerkstoff grundsätzlich als UV-härtbarer Lack, Acryllack und/oder Silikon-haltiger Lack ausgebildet sein. Im Fall der Ausgestaltung als UV-härtbarer Lack hat sich die zusätzliche Verwendung von Licht- und UV-stabilen Molybdän-/Wolfram-haltigen Verbindungen als vorteilhaft gezeigt, um Verfärbungen zu vermeiden. Umgekehrt können aber auch Licht- bzw. UV-labile Molybdän-/Wolfram-haltige Verbindungen verwendet und gleichzeitig mit der Aushärtung des Lacks umgewandelt werden. Verbundwerkstoff-Lacke auf Silikon-Basis besitzen aufgrund ihres geringen Anteils an organischen Gruppen den Vorteil einer sehr geringen Änderung ihres Filmvolumens während der Härtung. Hierdurch können sehr dichte Schichten mit guter Filmfestigkeit erzeugt werden, in denen die wenigstens eine Molybdän-/Wolfram-haltige Verbindung aufgenommen bzw. gehalten ist. Darüber hinaus weisen Silikon-Lacke eine hohe thermische Beständigkeit auf und eignen sich daher zur Beschichtung von Gegenständen, die zur Verwendung im Bereich von Wärmequellen vorgesehen sind.

Der Verbundwerkstoff kann zur Herstellung unterschiedlichster Produkte und Gegenstände verwendet werden. Das Produkt kann beispielsweise als Implantat, Katheter, Stent, Knochenimplantat, Zahnimplantat, Gefäßprothese, Endoprothese, E-xoprothese, Kabel, Schlauch, Lebensmittelverpackung, Behälter, Kraftstofftank, Haushaltsprodukt, Schalter, Armatur, Tastatur, Maus, Joystick, Gehäuse, Textil, Bekleidungsstück, Möbel- und/oder Innenausbauteil, Haushaltsgerät, Kreditkarte, Handygehäuse, Münze, Geldschein, Türschnalle, Kühlschrank, Rieselkörper im Kühlturm, Lackbeschichtung, Fliese oder ein Teil der Innenausstattung eines Gebäudes oder öffentlichen Verkehrsmittels ausgebildet sein. Weiterhin kann vorgesehen sein, dass der Verbundwerkstoff bzw. das Produkt als Lager- und Transportbehälter oder als Leitung für Kohlenwasserstoffe, Treibstoffe, Lösungsmittel und organische Flüssigkeiten ausgebildet ist. Ebenso kann der erfindungsgemäße Verbundwerkstoff zur Herstellung eines Gegenstands aus der Gruppe der Haushaltswaren, Medizintechnik, Lebensmitteltechnik, Sanitäranlagen, Verpackungen, Textilien, Klinken, Schalter, Sitze und Tastaturen verwendet werden. Ebenso eignet er sich für Produkte, die im häufigen Berührungskontakt mit Lebewesen stehen. Eine weitere vorteilhafte Verwendung liegt in Bauteilen für Klimaanlagen. Die Kühlrippen, die üblicherweise aus einer Cu- oder AI-Legierung bestehen, können in vorteilhafter Weise mit dem erfindungsgemäßen Verbundwerkstoff beschichtet oder aus diesem hergestellt werden. Auch die Schächte von Klimaanlagen in Gebäuden können antimikrobiell ausgeführt werden, indem der Verbundwerkstoff dem Schachtmaterial zugesetzt wird, das Schachtmaterial mit diesen beschichtet wird oder wenn das Schachtmaterial aus dem Verbundwerkstoff besteht. Auch Luftbefeuchter können mit entsprechenden antimikrobiellen Eigenschaften versehen werden. Außerdem kann der Verbundwerkstoff in Kabeln und/oder zur Herstellung von Kabeln verwendet werden.

Der Verbundwerkstoff kann als Beschichtungsmittel, insbesondere als Anstrichmittel, Lack und/oder Antifouling-Anstrich, ausgebildet sein. Unter einem Anstrichmittel werden Ausführungsformen des Verbundwerkstoffs verstanden, die eine flüssige bis pastenförmige Konsistenz besitzen und die auf Oberflächen aufgetragen einen physikalisch oder chemisch trocknenden Anstrich ergeben. Hierdurch können die vorteilhaften Eigenschaften des erfindungsgemäßen Verbundwerkstoffs besonders flexibel für beliebige Gegenstände und Oberflächen verwirklicht werden. Wichtige Ausgestaltungen sind beispielsweise Anti-Fouling Anstriche, z. B. für Schiffe, sowie antimikrobielle Ausstattung im Gesundheitswesen, der Industrie, dem Lebensmittelbereich und dem privaten Sektor.

In einem weiteren Ausführungsbeispiel wird zumindest die Oberfläche des Verbundwerkstoffs hydrophilisiert. Besonders vorteilhaft sind hier Hydrophilisierungsmittel (z.B. Irgasurf™ HL560, TechMer PPM15560™, Bayhydur™ 304) wie sie für PP-Textilfasern eingesetzt werden. Alternativ oder zusätzlich eignen sich Polyethylenglykol (PEG, PEG400), dessen Derivate, Hyaluronsäure, Stärke, oxethylierte Carbonsäureverbindungen, hydrophile Silikate, Atmer, Saccharosemethacrylate, hydrophiliertes, aliphatisches Polyisocyanat auf Basis von Hexamethylendiisocyanat (HDI) sowie diverse Fasern und GMS (Glyzerinmonostearat) sowie dessen Derivate. Weitere Stoffe für die Schaffung hydrophiler Eigenschaften sind Fettalkoholphosphate sowie Derivate von Polyethylenoxid (PEO), insbesondere mit Hydroxylendgruppen.

Unter hygroskopisch ist zu verstehen, dass das Verbundmaterial zumindest an seiner Oberfläche bzw. in den oberflächennahen Bereichen Feuchtigkeit aufnimmt. Beispielsweise sollte das Verbundmaterial in Umgebungen mit <10% relativer Luftfeuchtigkeit zwischen 0,01 bis 10 Gew.-% Feuchtigkeit aufnehmen. Besonders vorteilhaft sind 0,1 bis 3% Gleichgewichtsfeuchtigkeit, die sich in der Regel nach einigen Minuten bis Stunden einstellen.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, Einwaagefehlern, DIN-Toleranzen und dergleichen - als vom Rahmen der Erfindung mitumfasst anzusehen.

## Patentansprüche

1. Verfahren zum Herstellen eines dotierten Mischoxids für einen Verbundwerkstoff, wobei das Mischoxid die Summenformel MoₓW₁₋ₓM_{y}O_{z} besitzt, in welcher 0<x<1, 0,01 ≤y≤2 und 2,0≤z≤3,0 betragen und M ein von Mo und W verschiedenes Metall-Ion bezeichnet, bei welchem zumindest die Schritte:
- Lösen und/oder Suspendieren wenigstens einer Molybdänverbindung und wenigstens einer Wolframverbindung in wenigstens einem flüssigen Medium;
- Vermischen der wenigstens einen Molybdänverbindung und der wenigstens einen Wolframverbindung in einem vorbestimmten Massenverhältnis; und
- Trocknen der Mischung der wenigstens einen Molybdänverbindung und der wenigstens einen Wolframverbindung
durchgeführt werden, wobei zum Dotieren Fremdatome M ausgewählt aus der Gruppe Na, Cu, Bi, V, Ti und Zn in das Mischoxid eingebracht werden.

2. Verfahren nach Anspruch 1,
**dadurch gekennzeichnet, dass**
die wenigstens eine Molybdänverbindung ausgewählt wird aus einer Gruppe, die Ammoniumdimolybdat (ADM), Ammoniumparamolybdat (APM), Ammoniumpentamolybdat, Ammoniumheptamolybdat, Molybdänsäure, Molybdänoxidhydrate, Molybdänoxid, Molybdänsuboxid, metallisches Molybdän und Polyoxomolybdate umfasst und/oder dass die wenigstens eine Wolframverbindung ausgewählt wird aus einer Gruppe, die Ammoniummetawolframate (AMT), Wolframsäure, Wolframoxidhydrate, Wolframoxid, Wolframsuboxid, metallisches Wolfram und Polyoxowolframate umfasst.

3. Verfahren nach Anspruch 1 oder 2,
**dadurch gekennzeichnet, dass**
das Mischoxid mit einer Fluor-Verbindung, insbesondere mit einem Oxyfluorid, WOF₄, WO₂F₂, Calciumfluorid und/oder Fluorapatit, dotiert wird.

4. Verfahren nach einem der Ansprüche 1 bis 3,
**dadurch gekennzeichnet, dass**
das flüssige Medium ausgewählt wird aus einer Gruppe, die unpolare und/oder polare und/oder protische und/oder aprotische Lösungsmittel umfasst.

5. Verfahren nach einem der Ansprüche 1 bis 4,
**dadurch gekennzeichnet, dass**
die Lösung und/oder Suspension der wenigstens einen Molybdänverbindung und der wenigstens einen Wolframverbindung mit Hilfe wenigstens eines Verfahrens aus der Gruppe Sprühtrocknen, Gefriertrocknen, Verbrennungssynthese, Flammenhydrolyse, Gasphasensynthese und/oder Spraypyrolyse getrocknet werden.

6. Verfahren nach einem der Ansprüche 1 bis 5,
**dadurch gekennzeichnet, dass**
nach dem Trocknen wenigstens ein Kalzinierungsschritt und/oder wenigstens ein Zerkleinerungsschritt durchgeführt wird.

7. Verfahren nach Anspruch 6,
**dadurch gekennzeichnet, dass**
der Kalzinierungsschritt unter oxidierender und/oder reduzierender Atmosphäre und/oder unter Schutzgas und/oder bei Temperaturen zwischen 150°C und 1000°C durchgeführt wird und/oder dass der Zerkleinerungsschritt durch Trockenmahlung und/oder durch Strahlvermahlen und/oder bis zu einer mittleren Korngröße des Mischoxids von 0,1 µm bis 200 µm durchgeführt wird.

8. Verfahren nach einem der Ansprüche 1 bis 7,
**dadurch gekennzeichnet, dass**
das wenigstens eine Mischoxid zum Herstellen eines Verbundwerkstoffs in wenigstens einen weiteren Werkstoff eingearbeitet und/oder auf die Oberfläche des wenigstens einen weiteren Werkstoffs aufgebracht wird.

9. Verbundwerkstoff zur Herstellung antimikrobiell wirksamer Oberflächen, enthaltend wenigstens ein dotiertes Mischoxid, wobei das Mischoxid die Summenformel MoₓW₁₋ₓM_{y}O_{z} besitzt, in welcher 0<x<1, 0,01 ≤y≤2 und 2,0≤z≤3,0 betragen und M ein von Mo und W verschiedenes Metall-Ion bezeichnet, wobei als Dotierung Fremdatome M aus der Gruppe Na, Cu, Bi, V, Ti und Zn in das Mischoxid eingebracht sind.

10. Verbundwerkstoff nach Anspruch 9,
**dadurch gekennzeichnet,**
- **dass** x zwischen 0,50 und 0,70 beträgt; und/oder
- **dass** y zwischen 0,01 und 0,10 beträgt; und/oder
- **dass** z zwischen 2,50 und 3,0 beträgt; und/oder
- **dass** ein molares W:Mo-Verhältnis zwischen 250:1 und 1:250, insbesondere zwischen 3:1 und 1:3 beträgt.

11. Verbundwerkstoff nach Anspruch 9 oder 10,
**dadurch gekennzeichnet, dass**
dieser bezogen auf sein Gesamtgewicht einen Massenanteil zwischen 0,01 % und 80 %, insbesondere zwischen 0,1 % und 10 % an Mischoxid enthält.

12. Verbundwerkstoff nach einem der Ansprüche 9 bis 11,
**dadurch gekennzeichnet, dass**
das wenigstens eine Mischoxid in Form von Partikeln mit einem mittleren Durchmesser zwischen 0,1 µm und 200 µm, insbesondere zwischen 0,5 µm und 10 µm vorliegt.

13. Verbundwerkstoff nach einem der Ansprüche 9 bis 12,
**dadurch gekennzeichnet, dass**
dieser wenigstens einen weiteren Werkstoff umfasst, der ausgewählt ist aus einer Gruppe, die organische und anorganische Polymere, Kunststoffe, Silikone, Keramiken, Gummi, Pulverlacke, Flüssiglacke, Bitumen, Asphalt, Gläser, Wachse, Harze, Farben, Textilien, Gewebe, Holz, Kompositwerkstoffe, Metalle und Hydrophilierungsmittel und/oder ein Oxid von Zn, Bi, Cu, Ti und/oder V umfasst.

14. Verbundwerkstoff nach einem der Ansprüche 9 bis 13,
**dadurch gekennzeichnet, dass**
dieser zusätzlich zum dotierten Mischoxid wenigstens ein Molybdat und/oder wenigstens ein Wolframat und/oder eine Verbindung der Summenformel Aⁿ⁺_{z}MO₄, in welcher M Mo und/oder W bedeutet, A wenigstens ein von Mo und W verschiedenes Metall-Ion und/oder NH₄⁺ bezeichnet und n*z=+2 beträgt, enthält.

15. Verbundwerkstoff nach Anspruch 14,
**dadurch gekennzeichnet, dass**
A ausgewählt wird aus einer Gruppe, die Na, K, Mg, Ca, Ag, Cu, Bi, V, Ti und Zn umfasst.

## Claims

1. Method for preparing a doped mixed oxide for a composite material, wherein the mixed oxide has the molecular formula MoₓW₁₋ₓM_{y}O_{z}, in which 0 < x < 1, 0.01 ≤ y ≤ 2 and 2.0 ≤ z ≤ 3.0, and M denotes a metal ion that is different from Mo and W, in which method at least the following steps are carried out:
- dissolving and/or suspending at least one molybdenum compound and at least one tungsten compound in at least one liquid medium;
- mixing the at least one molybdenum compound and the at least one tungsten compound in a predetermined mass ratio; and
- drying the mixture of the at least one molybdenum compound and the at least one tungsten compound,
wherein, for doping, foreign atoms M selected from the group comprising Na, Cu, Bi, V, Ti and Zn are incorporated into the mixed oxide.

2. Method according to claim 1, **characterised in that** the at least one molybdenum compound is selected from a group comprising ammonium dimolybdate (ADM), ammonium paramolybdate (APM), ammonium pentamolybdate, ammonium heptamolybdate, molybdic acid, molybdenum oxide hydrates, molybdenum oxide, molybdenum suboxide, metallic molybdenum and polyoxomolybdates and/or **in that** the least one tungsten compound is selected from a group comprising ammonium metatungstates (AMT), tungstic acid, tungsten oxide hydrates, tungsten oxide, tungsten suboxide, metallic tungsten and polyoxotungstates.

3. Method according to either claim 1 or claim 2, **characterised in that** the mixed oxide is doped with a fluorine compound, in particular with an oxyfluoride, WOF₄, WO₂F₂, calcium fluoride and/or fluoroapatite.

4. Method according to any of claims 1 to 3, **characterised in that** the liquid medium is selected from a group that comprises non-polar and/or polar and/or protic and/or aprotic solvents.

5. Method according to any of claims 1 to 4, **characterised in that** the solution and/or suspension of the at least one molybdenum compound and the at least one tungsten compound are dried by means of at least one method selected from the group comprising spray drying, freeze drying, combustion synthesis, flame hydrolysis, gas phase synthesis and/or spray pyrolysis.

6. Method according to any of claims 1 to 5, **characterised in that** at least one calcination step and/or at least one comminution step is carried out after the drying.

7. Method according to claim 6, **characterised in that** the calcination step is carried out under an oxidising and/or reducing atmosphere and/or under protective gas and/or at temperatures of between 150°C and 1000°C and/or **in that** the comminution step is carried out by dry grinding and/or by jet milling and/or up to an average grain size of the mixed oxide of 0.1 µm to 200 µm.

8. Method according to any of claims 1 to 7, **characterised in that**, to prepare a composite material, the at least one mixed oxide is incorporated in at least one additional material and/or is applied to the surface of the at least one additional material.

9. Composite material for preparing antimicrobial surfaces, containing at least one doped mixed oxide, wherein the mixed oxide has the molecular formula MoₓW₁₋ₓM_{y}O_{z}, in which 0 < x < 1, 0.01 ≤ y ≤ 2 and 2.0 ≤ z ≤ 3.0, and M denotes a metal ion that is different from Mo and W, wherein, as doping, foreign atoms M selected from the group comprising Na, Cu, Bi, V, Ti and Zn are incorporated into the mixed oxide.

10. Composite material according to claim 9, **characterised in that**
- x is between 0.50 and 0.70; and/or
- y is between 0.01 and 0.10; and/or
- z is between 2.50 and 3.0; and/or
- a molar W:Mo ratio is between 250:1 and 1:250, in particular between 3:1 and 1:3.

11. Composite material according to either claim 9 or claim 10, **characterised in that** said material contains, based on the total weight thereof, a mass percentage of mixed oxide of between 0.01% and 80%, in particular between 0.1% and 10%.

12. Composite material according to any of claims 9 to 11, **characterised in that** there is at least one mixed oxide in the form of particles having an average diameter of between 0.1 µm and 200 µm, in particular of between 0.5 µm and 10 µm.

13. Composite material according to any of claims 9 to 12, **characterised in that** said material comprises at least one additional material selected from a group comprising organic and inorganic polymers, plastics materials, silicones, ceramics, rubber, coating powders, liquid paints, bitumen, asphalt, glasses, waxes, resins, dyes, textiles, woven fabrics, wood, composite materials, metals and hydrophilising agents and/or an oxide of Zn, Bi, Cu, Ti and/or V.

14. Composite material according to any of claims 9 to 13, **characterised in that** said material contains, in addition to the doped mixed oxide, at least one molybdate and/or at least one tungstate and/or a compound of molecular formula Aⁿ⁺_{z}MO₄, in which M denotes Mo and/or W, A denotes at least one metal ion that is different from Mo and W, and/or denotes NH₄⁺, and amounts to n*z=+2.

15. Composite material according to claim 14, **characterised in that** A is selected from a group comprising Na, K, Mg, Ca, Ag, Cu, Bi, V, Ti and Zn.

## Revendications

1. Procédé de production d'un oxyde mixte dopé pour un composite, dans lequel l'oxyde mixte dopé possède la formule brute MoₓW₁₋ₓM_{y}O_{z}, dans laquelle 0<x<1, 0,01≤y≤2 et 2,0≤z≤3,0 et M désigne un ion métallique différent de Mo et W, selon lequel on réalise au moins les étapes de :
- dissolution et/ou suspension d'au moins un composé de molybdène et au moins un composé de tungstène dans au moins un milieu liquide ;
- mélange du au moins un composé de molybdène et du au moins un composé de tungstène dans un rapport massique prédéfini ; et
- séchage du mélange du au moins un composé de molybdène et du au moins un composé de tungstène,
dans lequel des atomes étrangers M choisis dans le groupe Na, Cu, Bi, V, Ti et Zn sont introduits dans l'oxyde mixte pour le dopage.

2. Procédé selon la revendication 1,
**caractérisé en ce que**
le au moins un composé de molybdène est choisi dans un groupe comprenant le dimolybdate d'ammonium (ADM), le paramolybdate d'ammonium (APM), le pentamolybdate d'ammonium, l'heptamolybdate d'ammonium, l'acide molybdénique, l'oxyhydrate de molybdène, l'oxyde de molybdène, le sous-oxyde de molybdène, le molybdène métallique et les polyoxomolybdates et/ou **en ce que** le au moins un composé de tungstène est choisi dans un groupe comprenant les métatungstanates (AMT), l'acide tungstique, l'oxyhydrate de tungstène, l'oxyde de tungstène, le sous-oxyde de tungstène, le tungstène métallique et les polyoxotungstanates.

3. Procédé selon la revendication 1 ou 2,
**caractérisé en ce que**
l'oxyde mixte est dopé avec un composé fluoré, en particulier un oxyfluorure, le WOF₄, le WO₂F₂, le fluorure de calcium et/ou la fluoroapatite.

4. Procédé selon l'une des revendications 1 à 3,
**caractérisé en ce que**
le milieu liquide est choisi dans un groupe comprenant les solvants apolaires et/ou polaires et/ou protiques et/ou aprotiques.

5. Procédé selon l'une des revendications 1 à 4,
**caractérisé en ce que**
la solution et/ou la suspension du au moins un composé de molybdène et du au moins un composé de tungstène sont séchées à l'aide d'au moins un procédé provenant du groupe séchage par pulvérisation, séchage congélation, synthèse par combustion, hydrolyse à la flamme, synthèse en phase gazeuse et/ou pyrolyse par pulvérisation.

6. Procédé selon l'une des revendications 1 à 5,
**caractérisé en ce que**
après le séchage au moins une étape de calcination et/ou au moins une étape de broyage est réalisée.

7. Procédé selon la revendication 6,
**caractérisé en ce que**
l'étape de calcination est réalisée sous atmosphère oxydante et/ou réductrice et/ou sous gaz protecteur et/ou à des températures comprises entre 150 °C et 1000 °C et/ou **en ce que** l'étape de broyage est réalisée par mouture à sec et/ou par mouture à jets et/ou jusqu'à une granulométrie moyenne de l'oxyde mixte de 0,1 µm à 200 µm.

8. Procédé selon l'une des revendications 1 à 7,
**caractérisé en ce que**
le au moins un oxyde mixte est incorporé dans au moins un autre matériau pour la production d'un composite et/ou déposé sur la surface du au moins un autre matériau.

9. Composite pour la production de surfaces ayant une action anti-microbienne, contenant au moins un oxyde mixte dopé, dans lequel l'oxyde mixte possède la formule brute MoₓW₁₋ₓM_{y}O_{z}, dans laquelle 0<x<1, 0,01≤y≤2 et 2,0≤z≤3,0 et M désigne un ion métallique différent de Mo et W, dans lequel des atomes étrangers du groupe Na, Cu, Bi, V, Ti et Zn sont incorporés dans l'oxyde mixte.

10. Composite selon la revendication 9, **caractérisé en ce que**
- x est compris entre 0,50 et 0,70 ; et/ou
- y est compris entre 0,01 et 0,10 ; et/ou
- z est compris entre 2,50 et 3,0 ; et/ou
- un rapport molaire W/Mo est compris entre 250/1 et 1/250, en particulier entre 3/1 et 1/3.

11. Composite selon la revendication 9 ou 10,
**caractérisé en ce que**
celui-ci contient une proportion massique comprise entre 0,01 % et 80 %, en particulier entre 0,1 % et 10 % d'oxyde mixte par rapport à son poids total.

12. Composite selon l'une des revendications 9 à 11,
**caractérisé en ce que**
le au moins un oxyde mixte se présente sous forme de particules ayant un diamètre moyen de particules compris entre 0,1 µm et 200 µm, en particulier entre 0,5 µm et 10 µm.

13. Composite selon l'une des revendications 9 à 12,
**caractérisé en ce que**
celui-ci comprend au moins un autre matériau choisi dans un groupe comprenant les polymères organiques et inorganiques, les plastiques, les silicones, les céramiques, les caoutchoucs, les laques pulvérulentes, les laques liquides, les bitumes, l'asphalte, les verres, les cires, les résines, les peintures, les textiles, les tissus, le bois, les matériaux composites, les métaux et les agents hydrophilisants et/ou un oxyde de Zn, Bi, Cu, Ti et/ou V.

14. Composite selon l'une des revendications 9 à 13,
**caractérisé en ce que**
celui-ci comprend, en plus de l'oxyde mixte dopé, au moins un molybdate et/ou au moins un tungstanate et/ou un composé de formule brute Aⁿ⁺_{z}MO₄, dans laquelle M représente Mo et/ou W, A représente au moins un ion métallique différent de Mo et W, et/ou désigne NH₄⁺ et n*z=+2.

15. Composite selon la revendication 14,
**caractérisé en ce que**
A est choisi dans un groupe comprenant Na, K, Mg, Ca, Ag, Cu, Bi, V, Ti et Zn.
